# EUROPEAN PATENT SPECIFICATION

(11) **EP 3 643 251 B1**
(45) Date of publication and mention of the grant of the patent: **04.06.2025**
(21) Application number: 18821361.5
(22) Date of filing: 19.06.2018
(51) Int. Cl.: A61B 17/00, A61B 17/04, A61B 90/00

(54) **SUTURE DEVICE**
NAHTVORRICHTUNG
DISPOSITIF DE SUTURE

(30) Priority: 23.06.2017 JP 2017123283
(43) Date of publication of application: 29.04.2020
(73) Proprietor: Zeon Corporation, Tokyo 100-8246 (JP); National University Corporation Kagawa University, Takamatsu-shi, Kagawa 760-8521 (JP)
(72) Inventor: SUGITANI, Tatsurou, Tokyo 100-8246 (JP); KAWASAKI, Yojiro, Tokyo 100-8246 (JP); MIZUTANI, Kyoichiro, Tokyo 100-8246 (JP); MORI, Hirohito, Kita-gun Kagawa 761-0793 (JP)
(74) Representative: Maiwald GmbH
(86) International application number: PCT/JP2018/023335
(87) International publication number: WO 2018/235827

(56) References cited:
- JP-A- 2001 524 864
- JP-A- 2006 025 932
- JP-A- 2018 089 118
- US-A1- 2005 080 440
- US-A1- 2005 288 606
- US-A1- 2008 188 768
- US-A1- 2009 299 406
- US-A1- 2010 168 792
- US-A1- 2010 262 181
- US-A1- 2014 324 073

## Description

### TECHNICAL FIELD

The present invention relates to a suturing device suturing, for example, an incision formed in the lumen wall of a digestive tract or the like in natural orifice translumenal endoscopic surgery.

### BACKGROUND ART

Natural orifice translumenal endoscopic surgery (NOTES) has attracted attention in recent years as a minimally invasive surgery that does not create wounds on the body surface. Unlike conventional surgery in which the body surface is cut and an affected area in a body cavity is reached, in NOTES, an endoscope is used and a treatment tool reaches an affected area through the digestive tract, the urethra, the birth canal, or the like from a natural hole such as the mouth, the anus, the urethra, and the vaginal opening. As for such natural orifice translumenal endoscopic surgery, the possibility of application to various procedures has been reported, ranging from diagnostic procedures such as intraperitoneal observation and liver biopsy to appendectomy, cholecystectomy, fallopian tube ligation, ovariectomy, gastrointestinal anastomosis, and so on.

The suturing device that is described in Patent Document 1 below has been proposed as a suturing device suturing an incision (including a perforated part, a defective part, or the like) formed in the lumen wall of a digestive tract or the like in natural orifice translumenal endoscopic surgery. The suturing device is provided with a front side arm having bifurcated string support portions where a surgical string is attached so as to be passed over, a back side arm having a puncture needle, and a mechanism relatively pivoting and moving the arms.

A surgical string having engagement members attached to both end portions is used as a surgical string used in such a suturing device. The surgical string is supported so as to be detachable between the bifurcated string support portions of the front side arm by the engagement members at both ends of the surgical string being respectively attached to the string support portions. In this state, the back side arm is brought close to the front side arm, the distal end portion of the needle pierces the engagement member of the surgical string, and the engagement member is engaged with the distal end portion of the needle. Subsequently, the back side arm is separated from the front side arm. As a result, the engagement member engaged with the distal end portion of the needle is separated from the corresponding string support portion.

By the way, in a state where the distal end portion of the needle is exposed, the distal end of the needle may be caught at an unexpected part and insertion into a lumen in the body may be hindered during the insertion. Conceivable as a structure for preventing this is a structure in which a third arm (needle accommodating arm) having a needle accommodating hole is provided at the part between the pair of string support portions of the front side arm or the width of one string support portion is increased for a needle accommodating hole to be formed adjacent to an accommodating portion accommodating the engagement member of the surgical string and the needle accommodating hole to be capable of accommodating the distal end portion of the needle. However, in these measures, the enlarged part of the needle accommodating arm or the string support portion may act as a hindrance and obstruct treatment.

### CITATION LIST

### PATENT DOCUMENT

Patent Document 1: WO 2012/101999 A

JP 2018 089118 A describes a medical treatment instrument for carrying out various treatments to a body tissue through a transluminal cavity using an endoscope.

### SUMMARY OF THE INVENTION

### PROBLEM TO BE SOLVED BY THE INVENTION

The invention has been made in view of such a situation, and an object of the invention is to provide a suturing device with which insertion into the body can be smoothly carried out without treatment being hindered.

### MEANS FOR SOLVING PROBLEM

In order to achieve the above objective, a suturing device according to the invention as defined by the features of the independent claim is provided.

According to the invention, the slide regulation means is provided and the slide regulation means releasably regulates the sliding of the first operation portion and the second operation portion in a state where the relative positions of the first operation portion and the second operation portion are adjusted for the halted state where the distal end portion of the needle-shaped member has approached the engagement member accommodated in the engagement member accommodating portion without engagement with the engagement member. In the halted state, the distal end of the needle-shaped member is in the engagement member accommodating portion of the string support portion of the second arm or at a position close to the engagement member accommodating portion. Accordingly, when the suturing unit is inserted into a body, the distal end of the needle-shaped member is prevented from interfering with a lumen wall or another part. In addition, such a function is realized only in the operation unit without any change in the configuration in the suturing unit. Accordingly, the insertion into the body can be smoothly carried out without treatment being hindered.

In the suturing device according to the invention, the slide regulation means is capable of having a first projection portion provided on the first operation portion, a second projection portion provided on the second operation portion so as to correspond to the first projection portion, and a fixing member and the fixing member is capable of having a first fitted portion having one end portion into which a part of the first projection portion is detachably fitted in the halted state, a second fitted portion having the other end portion into which a part of the second projection portion is detachably fitted in the halted state, and a connecting portion integrally connecting the first fitted portion and the second fitted portion.

When the suturing unit is inserted into a body, the fixing member is attached to the operation unit by a part of the first projection portion being fitted into the first fitted portion and a part of the second projection portion being fitted into the second fitted portion. As a result, the sliding of the first operation portion and the second operation portion can be regulated in the halted state. When a suture treatment is performed by the suturing unit being inserted into a body, the regulation of the sliding of the first operation portion and the second operation portion is released by the fixing member being removed, and thus a necessary operation can be carried out.

In the suturing device according to the invention, the slide regulation means is capable of having a first through hole portion provided in the first operation portion, a second through hole portion provided in the second operation portion so as to correspond to the first through hole portion in the halted state, and a pin member inserted in an insertable and removable manner into the first through hole portion and the second through hole portion in a state where the first through hole portion and the second through hole portion correspond to each other.

When the suturing unit is inserted into a body, the pin member is inserted into the first through hole portion of the first operation portion and the second through hole portion of the second operation portion disposed relative to each other. As a result, the sliding of the first operation portion and the second operation portion can be regulated in the halted state. When a suture treatment is performed by the suturing unit being inserted into a body, the regulation of the sliding of the first operation portion and the second operation portion is released by the pin member being removed, and thus a necessary operation can be carried out.

In the suturing device according to the invention, the slide regulation means is capable of having a clip member pivotably provided on one of the first operation portion and the second operation portion and an engaged portion provided in the other of the first operation portion and the second operation portion, a distal end portion of the clip member releasably engaging with the engaged portion, and the distal end portion of the clip member is capable of engaging with the engaged portion by the clip member being pivoted in a predetermined direction in the halted state and the engagement of the distal end portion of the clip member with the engaged portion is releasable by the clip member being pivoted in a direction opposite to the predetermined direction.

When the suturing unit is inserted into a body, the distal end portion of the clip member is engaged with the engaged portion by the clip member being pivoted in a predetermined direction. As a result, the sliding of the first operation portion and the second operation portion can be regulated in the halted state. When a suture treatment is performed by the suturing unit being inserted into a body, the engagement of the distal end portion of the clip member with the engaged portion is released by the clip member being pivoted in the direction opposite to the predetermined direction. As a result, the regulation of the sliding of the first operation portion and the second operation portion is released, and thus a necessary operation can be carried out.

### BRIEF DESCRIPTION OF DRAWINGS

Fig. 1A is a front view illustrating the configuration of an operation unit of a suturing device according to an embodiment of the invention;
Fig. 1B is a front view illustrating the configuration of a suturing unit of the suturing device according to the embodiment of the invention;
Fig. 2A is a front view of a back side arm constituting the suturing unit illustrated in Fig. 1B;
Fig. 2B is a plan view of the back side arm illustrated in Fig. 2A;
Fig. 3A is a front view of a front side arm constituting the suturing unit illustrated in Fig. 1B;
Fig. 3B is a plan view of the front side arm illustrated in Fig. 3A;
Fig. 3C is a plan view illustrating a state where a cap of the front side arm illustrated in Fig. 3B is removed;
Fig. 4A is an enlarged partial cross-sectional view illustrating a main portion of the suturing unit illustrated in Fig. 1B;
Fig. 4B is an enlarged partial cross-sectional view illustrating the main portion of the suturing unit illustrated in Fig. 1B;
Fig. 5A is a front view for describing the operation of the back side arm with respect to the front side arm and is a view illustrating a state where the back side arm is separated from the front side arm;
Fig. 5B is a plan view of Fig. 5A;
Fig. 6A is a front view for describing the operation of the back side arm with respect to the front side arm and is a view illustrating a state where a needle-shaped member is positioned in one string support portion and the back side arm is brought close to the front side arm;
Fig. 6B is a plan view of Fig. 6A;
Fig. 7A is a front view for describing the operation of the back side arm with respect to the front side arm and is a view illustrating a state where the needle-shaped member is positioned in the other string support portion and the back side arm is brought close to the front side arm;
Fig. 7B is a plan view of Fig. 7A;
Fig. 8A is a longitudinal cross-sectional view of the operation unit illustrated in Fig. 1A ;
Fig. 8B is a cross-sectional view taken along line VIIIb-VIIIb in Fig. 1A ;
Fig. 8C is a perspective view of a fixing holder constituting the operation unit illustrated inFig. 1A ;
Fig. 8D is a perspective view illustrating another configuration of the fixing holder constituting the operation unit illustrated in Fig. 1A ;
Fig. 9A is a cross-sectional view taken along line VIIIb-VIIIb inFig. 1A and is a view illustrating a state where the fixing holder is attached;
Fig. 9B is a cross-sectional view illustrating an example of the operation at a time when the fixing holder is removed from the state that is illustrated in Fig. 9A;
Fig. 9C is a cross-sectional view illustrating another example of the operation at a time when the fixing holder is removed from the state that is illustrated in Fig. 9A;
Fig. 10A is a front view illustrating the configuration of an operation unit of a suturing device according to another embodiment of the invention;
Fig. 10B is a cross-sectional view taken along line Xb-Xb in Fig. 10A;
Fig. 10C is a front view illustrating a state where a pin member of the operation unit illustrated in Fig. 10A is removed;
Fig. 10D is a front view illustrating a state where a slider portion is slid in a direction of pulling with respect to a base portion from the state that is illustrated in Fig. 10A;
Fig. 10E is a front view illustrating a state where the slider portion is slid in a direction of pushing with respect to the base portion from the state that is illustrated in Fig. 10A;
Fig. 11A is a front view illustrating the configuration of an operation unit of a suturing device according to yet another embodiment of the invention;
Fig. 11B is a front view illustrating a state where engagement is released by a clip member of the operation unit illustrated in Fig. 11A being pivoted;
Fig. 11C is a front view illustrating a state where a slider portion is slid in the direction of pulling with respect to a base portion from the state that is illustrated in Fig. 11B;
Fig. 11D is a front view illustrating a state where the slider portion is slid in the direction of pushing with respect to the base portion from the state that is illustrated in Fig. 11B;
Fig. 12A is a view illustrating the procedure of a procedure (suturing) performed by means of the suturing device according to the embodiment of the invention and is a view illustrating a first process;
Fig. 12B is a view illustrating a second process subsequent to Fig. 12A;
Fig. 12C is a view illustrating a third process subsequent to Fig. 12B;
Fig. 12D is a view illustrating a fourth process subsequent to Fig. 12C;
Fig. 12E is a view illustrating a fifth process subsequent to Fig. 12D;
Fig. 12F is a view illustrating a sixth process subsequent to Fig. 12E;
Fig. 12G is a view illustrating a seventh process subsequent to Fig. 12F;
Fig. 12H is a view illustrating an eighth process subsequent to Fig. 12G;
Fig. 12I is a view illustrating a ninth process subsequent to Fig. 12H;
Fig. 12J is a view illustrating a tenth process subsequent to Fig. 12I; and
Fig. 12K is a view illustrating a final process subsequent to Fig. 12J.

### MODE(S) FOR CARRYING OUT THE INVENTION

Hereinafter, an embodiment of the invention will be described in detail with reference to the drawings.

### (Overall Configuration of Suturing Device)

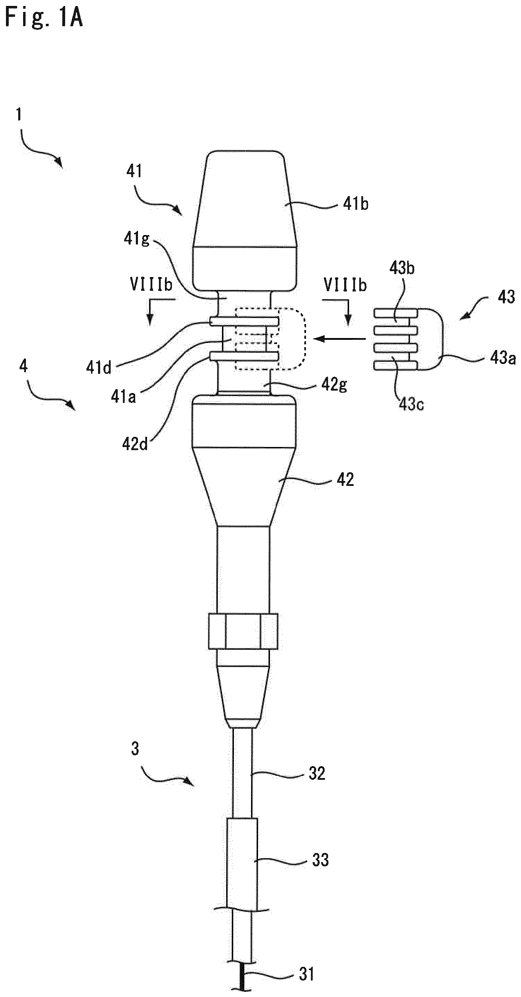

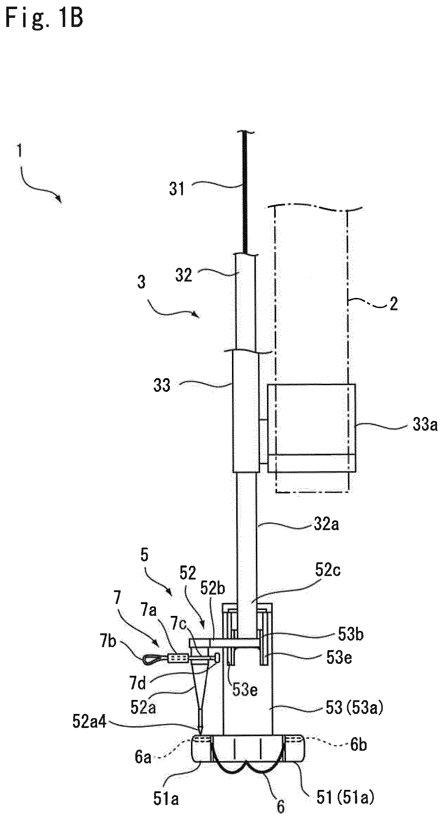

As illustrated in Fig. 1A and 1B, a suturing device 1 according to the present embodiment is a device that is inserted into a body in a state where the suturing device 1 is attached to an endoscope (such as a flexible endoscope) 2 and is a device for suturing, for example, an incision formed in a digestive tract or an organ in an abdominal cavity by an operation outside the body while performing visual recognition by means of a camera (not illustrated) of the endoscope 2.

Schematically, the suturing device 1 is configured to be provided with a sheath unit 3 disposed along the shaft of the endoscope 2, an operation unit (handle) 4 provided at the proximal end of the sheath unit 3, and a suturing unit 5 provided at the distal end of the sheath unit 3.

### (Sheath Unit)

The sheath unit 3 is a long member extending along an axial direction. Two tubes and one wire (or three tubes) flexible enough to bend to follow the bending of the shaft of the endoscope 2 constitute the sheath unit 3. In other words, schematically, the sheath unit 3 is configured to be provided with a front side arm moving wire (first long member) 31, a back side arm moving tube (second long member) 32, and a case tube 33.

The front side arm moving wire 31 is made of a long and flexible wire rod, is slidably inserted through the back side arm moving tube 32, and is provided in the back side arm moving tube 32 so as to be movable along the axial direction and rotatable around an axis. In the present embodiment, a wire rope is used as the front side arm moving wire 31. The wire rope is a rope made of a stranded wire formed by a plurality of wires (cables) made of metal (stainless steel) or the like being twisted in a spiral shape. However, a wire made of a single wire may also be used as the wire 31. In addition, a hollow tube may be used instead of the wire 31.

The back side arm moving tube 32 is made of a long hollow tube that is slidably inserted through the case tube 33 and is provided in the case tube 33 so as to be movable along the axial direction and rotatable around the axis. Although a flexible tube made of resin or the like may be used as the back side arm moving tube 32, a highly durable coil tube is used as the back side arm moving tube 32 in the present embodiment. A flat wire coil tube formed by a long flat plate made of metal (stainless steel) or the like being wound in a spiral shape can be used as the coil tube. However, a round wire coil tube or a coil tube with a flat inner surface may also be used as the coil tube.

It should be noted that a wire tube may be used as the back side arm moving tube 32. The wire tube is a tube formed by a plurality of wires (cables) made of metal (stainless steel) or the like being twisted in a spiral shape so as to become hollow.

Although the back side arm moving tube 32 may be a member (such as a coil tube) that is uniform from the proximal end of the back side arm moving tube 32 to the distal end of the back side arm moving tube 32, a distal end portion (tube distal end portion) 32a of the back side arm moving tube 32 in the present embodiment is formed by a substantially cylindrical member made of a highly rigid metal being welded and fixed to the distal end of the coil tube.

The case tube 33 is a flexible hollow tube made of resin or the like. The proximal ends of the front side arm moving wire 31 and the back side arm moving tube 32 are connected to the operation unit 4 and the distal ends of the front side arm moving wire 31 and the back side arm moving tube 32 are connected to the suturing unit 5. The proximal end of the case tube 33 reaches the vicinity of the operation unit 4 without being connected to the operation unit 4 and the distal end of the case tube 33 reaches the vicinity of the suturing unit 5 without being connected to the suturing unit 5. The case tube 33 can be formed of a material such as polyethylene and vinyl chloride.

A substantially cylindrical fixing member 33a is integrally attached to the distal end of the case tube 33. The suturing device 1 can be detachably fixed to the endoscope 2 by the distal end portion of the shaft of the endoscope 2 being press-fitted into the fixing member 33a. It is possible to move the entire suturing unit 5 with respect to the endoscope 2 by moving (sliding) the entire operation unit 4 to the distal end side or the proximal end side with respect to the case tube 33. It is possible to rotate the entire suturing unit 5 by rotating the entire operation unit 4 around the axis with respect to the case tube 33.

### (Suturing Unit)

The suturing unit 5 of the suturing device 1 is configured as illustrated in Fig. 1B. In other words, the suturing unit 5 is configured to be provided with a front side arm (first arm) 51 to which a surgical string 6 is attached and a back side arm (second arm) 52 having a needle-shaped member 52a.

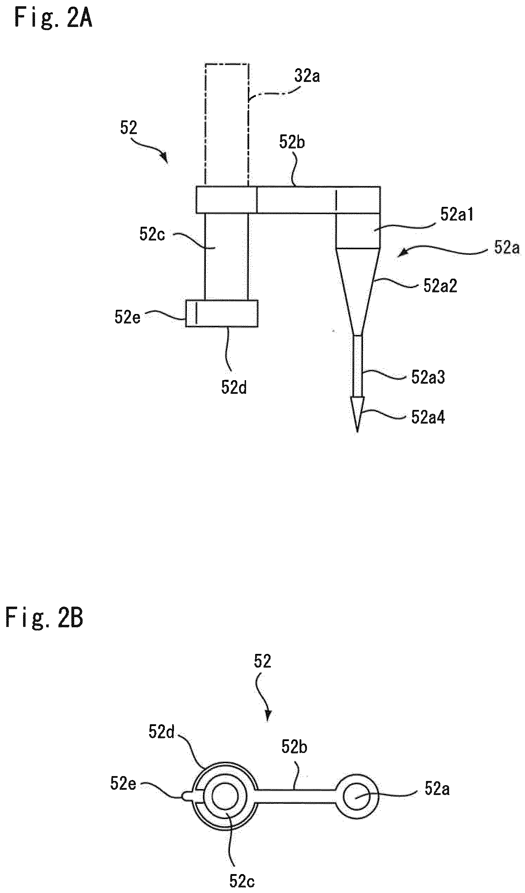

As illustrated in Figs. 2A and 2B, the back side arm 52 has the needle-shaped member 52a, a needle support portion 52b, a back side guide portion 52c, and a projection member 52d. The back side guide portion 52c is made of a substantially cylindrical member. One end of the substantially strip-shaped needle support portion 52b is fixed to the upper end side of the back side guide portion 52c. The projection member 52d is fixed to the lower end side of the back side guide portion 52c.

The proximal end portion of the needle-shaped member 52a is fixed to the other end of the needle support portion 52b so as to be directed forward (to the distal end side). The projection member 52d has a projection portion 52e. Although not particularly limited, the needle-shaped member 52a, the needle support portion 52b, the back side guide portion 52c, and the projection member 52d are made of metal such as stainless steel and are integrally fixed to each other by laser welding or the like after being formed as separate components. It should be noted that the projection portion 52e in the present embodiment is disposed so as to protrude to the side that is 180° opposite to the direction of extension of the needle support portion 52b although the projection portion 52e is not particularly limited.

As for the back side arm 52, the rear end surface (end surface on the proximal end side) of the back side guide portion 52c is integrally fixed by laser welding or the like to the distal end portion 32a of the back side arm moving tube 32 (see Fig. 1B). The back side guide portion 52c is inserted into a front side guide portion 53 (described later) from the end portion of the back side guide portion 52c that is on the projection member 52d side and is held so as to be slidable along the axial direction and pivotable around the axis.

The needle-shaped member 52a has a straight body-shaped large-diameter portion 52a1, a tapered tapered portion 52a2, a straight body-shaped small-diameter portion 52a3, and an arrowhead portion 52a4 from the proximal end side of the needle-shaped member 52a toward the distal end side of the needle-shaped member 52a. The arrowhead portion 52a4 is formed in a tapered tapered shape and has a sharp distal end. The arrowhead portion 52a4 is formed such that the outer diameter of the proximal end of the arrowhead portion 52a4 is larger than the outer diameter of the distal end of the small-diameter portion 52a3 and the connecting part between the arrowhead portion 52a4 and the small-diameter portion 52a3 is slightly stepped. The needle-shaped member 52a is fixed to the needle support portion 52b such that the central axis of the needle-shaped member 52a is substantially parallel to the central axis of the back side guide portion 52c with the distal end of the needle-shaped member 52a facing the front side arm 51.

By rotating the back side arm moving tube 32 around the central axis of the back side arm moving tube 32, it is possible to pivot (turn) the needle-shaped member 52a around the central axis of the tube distal end portion 32a while maintaining a state where the central axis of the needle-shaped member 52a is substantially parallel to the central axis of the tube distal end portion 32a.

It should be noted that the needle-shaped member 52a may have a length and strength at which the needle-shaped member 52a is capable of piercing a suture target (lumen wall), penetrating the target, and being pulled out from the target by being moved in the opposite direction with the target penetrated and is not particularly limited in terms of material, length, and shaft diameter. For example, in a case where a stomach wall is sutured by means of the suturing device 1, the length of the needle-shaped member 52a may be a length at which the needle-shaped member 52a is capable of penetrating the stomach wall and it is preferable in terms of strength that the material of the needle-shaped member 52a is metallic. For example, the length of the needle-shaped member 52a is preferably approximately 7 to 20 mm and more preferably approximately 7 to 10 mm. In addition, it is preferable that the shaft diameter of the large-diameter portion 52a1 of the needle-shaped member 52a is approximately 1.5 to 3.0 mm, the shaft diameter of the small-diameter portion 52a3 is approximately 0.5 to 1 mm, and the maximum shaft diameter of the arrowhead portion 52a4 is approximately 0.6 to 1.5 mm.

As illustrated in Fig. 1B, a ligation tool 7 for ligating the surgical string 6 is attached to the proximal end portion (large-diameter portion 52a1) of the needle-shaped member 52a. The ligation tool 7 is configured by a substantially cylindrical tube (tightening tube) 7a made of an elastic material being slidably and externally fitted to a connecting portion and a main body portion in which a ligation loop 7c through which the surgical string 6 is passed is integrally provided at one axial end of the connecting portion formed in a substantially columnar shape and a connecting loop 7b to which a ligation device is connected is integrally provided at the other axial end. During the ligation, the ligation loop 7c is drawn into the lumen by the tube 7a being slid to the ligation loop 7c side. Polyamide resin or the like is used as the material of the main body portion and a silicone elastomer or the like is used as the material of the tightening tube 7a.

It should be noted that a locking member 7d locking a part of the ligation loop 7c is provided in the present embodiment and, although not particularly limited, the locking member 7d is provided so that the ligation loop 7c is prevented from being excessively drawn into the tube 7a and the ligation loop 7c is prevented from escaping from the tube 7a. In addition, a ligation tool having a configuration in which a loop member is formed by both ends of a flexible wire rod being coupled and molding into a substantially loop shape (endless shape) being performed, the intermediate parts of the loop member are brought close to each other so as to be substantially parallel, and the substantially cylindrical tube (tightening tube) 7a made of an elastic material is slidably and externally fitted to the intermediate parts may be used as the ligation tool 7.

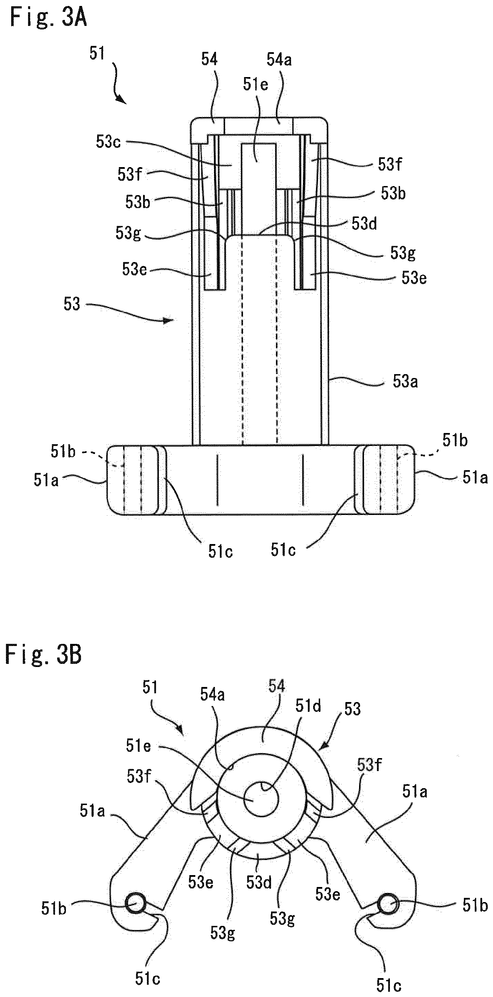

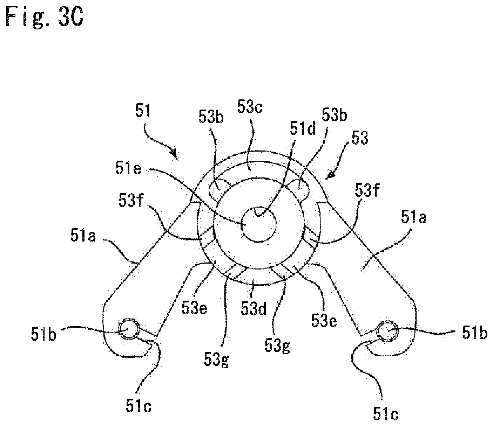

As illustrated in Figs. 1B and 3A to 3C, schematically, the front side arm 51 is configured to be provided with a pair of string support portions 51a and 51a, the front side guide portion 53, and a cap 54. The pair of string support portions 51a and 51a are formed in a substantially V shape (which may be a circular arc shape, a squared U shape, a U shape, or the like) and are bifurcated. The surgical string 6 is attached so as to extend between the vicinities of the respective distal end portions of the string support portions 51a and 51a. Engagement members 6a and 6b are attached to both ends of the surgical string 6 and each of the engagement members 6a and 6b is formed in an annular shape (see Fig. 1B).

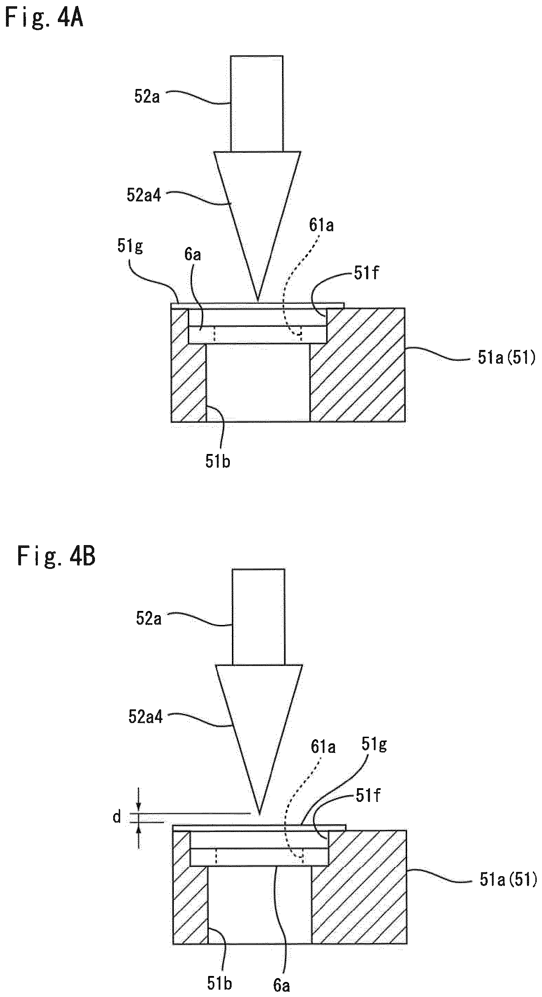

As illustrated in Fig. 4A, through holes 51b and 51b penetrating the respective distal end portions of the string support portions 51a and 51a of the front side arm 51 across front surfaces (surfaces on the distal end side) and rear surfaces (surfaces on the proximal end side) are formed in the respective distal end portions of the string support portions 51a and 51a of the front side arm 51 and an accommodating space (engagement member accommodating portion) 51f where the engagement members 6a and 6b are engageable is formed on the rear surface side of the through holes 51b and 51b. A through groove 51c is formed inside and outside at a part of the side portion of each of the through holes 51b and 51b so as to allow the surgical string 6 to pass. The accommodating space 51f is slightly larger in diameter than the through holes 51b and 51b and is set to a diameter at which the engagement members 6a and 6b can be releasably engaged by the inner wall of the accommodating space 51f in a state where a through hole 61a penetrating the front and back of the engagement members 6a and 6b is disposed so as to be substantially concentric with the through holes 51b and 51b of the string support portions 51a and 51a.

The through hole 61a of each of the engagement members 6a and 6b has a structure in which the engagement members 6a and 6b are not detached from the needle-shaped member 52a when the arrowhead portion 52a4 is completely inserted through the through hole 61a although the arrowhead portion 52a4 of the needle-shaped member 52a can be inserted through the through hole 61a. Specifically, each of the engagement members 6a and 6b is formed such that the inner diameter of each of the engagement members 6a and 6b is smaller than the outer diameter of the arrowhead portion 52a4 of the needle-shaped member 52a and larger than the shaft diameter of the distal end (that is, the part of connection to the arrowhead portion 52a4) of the small-diameter portion 52a3 of the needle-shaped member 52a. It should be noted that a surgical string accommodating portion (not illustrated) is provided in the middle portions of the string support portions 51a and 51a and the intermediate part of the surgical string 6 disposed across the string support portions 51a and 51a is accommodated in the surgical string accommodating portion.

An engagement member fixing film 51g for reliably preventing any unintended falling of the engagement members 6a and 6b accommodated in the accommodating space 51f is pasted on the rear surface (upper surface in Fig. 4A) side of the accommodating space 51f of each of the string support portions 51a and 51a. A thin film having a thickness of approximately 0.04 mm and formed of resin such as polypropylene as a material constitutes the engagement member fixing film 51g such that the engagement member fixing film 51g is easily pierced by the arrowhead portion 52a4 of the needle-shaped member 52a.

The joining part between the respective proximal end portion sides of the string support portions 51a and 51a of the front side arm 51 has a through hole 51d penetrating the joining part upward and downward. A substantially columnar guide shaft 51e is fixed to the through hole 51d so as to protrude toward the rear surface side. The guide shaft 51e is a member that is slidably inserted into the lumen of the back side guide portion 52c and guides the reciprocating movement and pivoting of the back side arm 52 in the central axis direction. In the present embodiment, the guide shaft 51e is made of metal such as stainless steel and the distal end of the front side arm moving wire 31 is integrally fixed by laser welding or the like to the rear end (end portion on the proximal end side) of the guide shaft 51e.

The front side guide portion 53 is made of a substantially cylindrical member. A thick portion 53a of the front side guide portion 53 is formed by the thickness of a part on the back surface side exceeding the thickness of the front surface side. A pair of recessed guide grooves 53b and 53b are formed in the thick portion 53a so as to extend in the upward-downward direction (direction substantially parallel to the central axis) and be recessed in a radiation direction. The recessed guide grooves 53b and 53b are parts guiding the sliding of the back side arm 52 in the central axis direction by the projection portion 52e formed on the projection member 52d of the back side arm 52 being slidably and loosely fitted into either of the recessed guide grooves 53b and 53b.

The recessed guide grooves 53b and 53b do not reach the upper portion (rear end) of the front side guide portion 53. A connection groove 53c that is recessed in a circular arc shape is formed in the upper portion of the front side guide portion 53. The connection groove 53c is a part for sliding the back side guide portion 52c to the proximal end side with respect to the front side guide portion 53 and allowing the back side guide portion 52c (back side arm 52) to turn (pivot) when the projection portion 52e is at the position of the connection groove 53c (that is, in a state where the projection portion 52e has escaped from the recessed guide grooves 53b and 53b). Both ends (surfaces) of the connection groove 53c are respectively continuous with the outside parts of the inner walls of the recessed guide grooves 53b and 53b and have a depth equal to the depth of the recessed guide grooves 53b and 53b. It should be noted that the recessed guide grooves 53b and 53b reach the lower portion (front end) of the front side guide portion 53.

A notch portion is formed in the upper portion of the front side guide portion 53 that is on the front surface side. Formed on both sides of a bottom side portion (side portion on the front end side) 53d of the notch portion are through guide grooves 53e and 53e extending in the upward-downward direction (direction substantially parallel to the central axis) and formed for inside and outside penetration. The through guide grooves 53e and 53e are parts guiding the sliding of the back side arm 52 in the central axis direction by the needle support portion 52b of the back side arm 52 being slidably and loosely fitted into either of the through guide grooves 53e and 53e. The side portions on both sides of the through guide grooves 53e and 53e are surfaces substantially parallel to the central axis direction and substantially parallel to each other.

The upper ends (rear ends) of the through guide grooves 53e and 53e reach the bottom side portion 53d of the notch portion. The lower ends (front ends) of the through guide grooves 53e and 53e do not reach the lower portion (front end) of the front side guide portion 53. The positions of the lower ends (front ends) of the through guide grooves 53e and 53e are set to positions at which the rear end of the arrowhead portion 52a4 of the needle-shaped member 52a is capable of penetrating the engagement member 6a or 6b of the surgical string 6 and engaging when the needle support portion 52b of the front side arm 51 has been fitted and reached the lower end.

Inclined portions 53f and 53f are parts of both side portions of the notch portion of the front side guide portion 53 that are on the rear side (proximal end side) and the remaining part that is on the front side (distal end side) is formed so as to be substantially the same surface as ones of both side portions of the through guide grooves 53e and 53e (sides that are mutually outside). The connection parts between the bottom side portion 53d and the others of both side portions of the through guide grooves 53e and 53e (sides that are mutually inside) are chamfered in a circular arc shape as chamfered portions 53g and 53g such that the needle support portion 52b is smoothly guided into the through guide grooves 53e and 53e. It should be noted that this connection part is not limited to the circular arc shape and may have an inclined surface shape on condition that the needle support portion 52b is smoothly guided into the through guide grooves 53e and 53e.

The cap 54 is attached to the rear end of the front side guide portion 53. The cap 54 has a circular arc-shaped recessed portion 54a where the back side guide portion 52c is disposed. The cap 54 is a member that prevents the back side arm 52 from escaping from the front side arm 51 by being attached to the rear end of the front side guide portion 53 in a state where the shaft pin 51e is inserted in the back side guide portion 52c and the back side arm 52 and the front side arm 51 are combined.

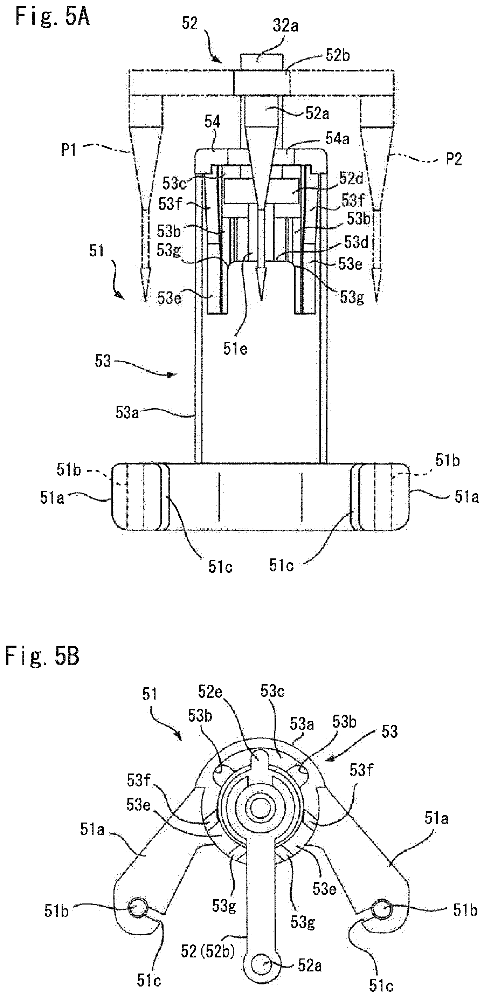

Next, the relative operations of the front side arm 51 and the back side arm 52 will be described with reference to Figs. 5A, 5B, 6A, 6B, 7A, and 7B. First, as illustrated in Figs. 5A and 5B, the projection member 52d (projection portion 52e) of the back side arm 52 is positioned in the connection groove 53c of the front side guide portion 53 in a state where the back side arm 52 is slid so as to be separated from the front side arm 51. In this state, the back side arm 52 is capable of freely pivoting (turning) within a predetermined angle range defined by both end surfaces of the connection groove 53c (approximately 90° in the present embodiment) around the central axis with respect to the front side arm 51.

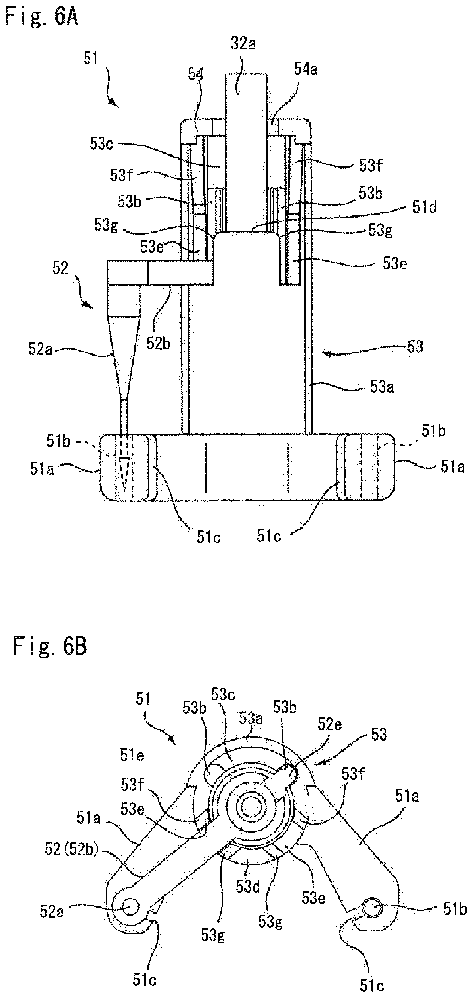

In this state, the back side arm 52 is pivoted as illustrated with the one-dot chain line indicated by Sign P1 in Fig. 5A (clockwise in Fig. 5B). Then, the projection portion 52e of the back side arm 52 abuts against one end surface of the connection groove 53c of the front side guide portion 53 and further clockwise pivoting is regulated (see Fig. 6B). In this state, the back side arm 52 is slid so as to approach the front side arm 51. Then, the projection portion 52e moves along one end surface of the connection groove 53c and the projection portion 52e is guided to one recessed guide groove 53b of the front side guide portion 53 and fitted (loosely fitted) into the recessed guide groove 53b. The fitting between the projection portion 52e and the recessed guide groove 53b is set relatively rough such that the projection portion 52e is smoothly guided to the recessed guide groove 53b. In other words, a relatively large clearance is set.

As a result, the pivoting of the back side arm 52 with respect to the front side arm 51 is limited and the back side arm 52 is guided in the central axis direction along the recessed guide groove 53b. When the back side arm 52 is slid so as to further approach the front side arm 51, the needle support portion 52b is guided to the through guide groove 53e and fitted (loosely fitted) into the through guide groove 53e. The fitting between the needle support portion 52b and the through guide groove 53e is set relatively tight on condition that the sliding is not hindered such that the distal end of the arrowhead portion 52a4 of the needle-shaped member 52a is accurately guided to the through hole 51b of one string support portion 51a. In other words, a relatively small clearance is set.

When the back side arm 52 is slid so as to further approach the front side arm 51, the needle support portion 52b is guided to the through guide groove 53e, the distal end of the arrowhead portion 52a4 of the needle-shaped member 52a is guided to the through hole 51b of one string support portion 51a, and the needle support portion 52b abuts against the front end of the through guide groove 53e and stops. Performed as a result as illustrated in Figs. 6A and 6B is positioning at a predetermined position where the distal end of the arrowhead portion 52a4 of the needle-shaped member 52a can be appropriately engaged with one engagement member 6a (see Fig. 1B) of the surgical string 6 supported in the accommodating space 51f (see Fig. 4A) of one string support portion 51a.

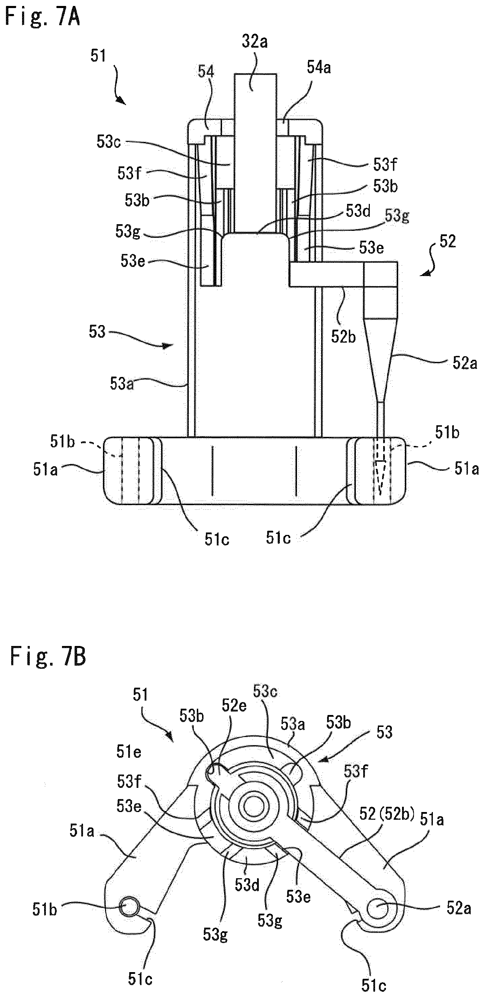

Next, as illustrated with the one-dot chain line indicated by Sign P1 in Fig. 5A, the back side arm 52 is returned to the original state by being slid so as to be separated from the front side arm 51 and the projection member 52d (projection portion 52e) of the back side arm 52 is positioned in the connection groove 53c of the front side guide portion 53. In this state, the back side arm 52 is pivoted as illustrated with the two-dot chain line indicated by Sign P2 in Fig. 5A (counterclockwise in Fig. 5B). Then, the projection portion 52e of the back side arm 52 abuts against the other end surface of the connection groove 53c of the front side guide portion 53 and further counterclockwise pivoting is regulated (see Fig. 7B). In this state, the back side arm 52 is slid so as to approach the front side arm 51. Then, the projection portion 52e moves along the other end surface of the connection groove 53c and the projection portion 52e is guided to the other recessed guide groove 53b of the front side guide portion 53 and fitted (loosely fitted) into the recessed guide groove 53b. The fitting between the projection portion 52e and the recessed guide groove 53b is set relatively rough such that the projection portion 52e is smoothly guided to the recessed guide groove 53b. In other words, a relatively large clearance is set.

As a result, the pivoting of the back side arm 52 with respect to the front side arm 51 is limited and the back side arm 52 is guided in the central axis direction along the recessed guide groove 53b. When the back side arm 52 is slid so as to further approach the front side arm 51, the needle support portion 52b is guided to the through guide groove 53e and fitted (loosely fitted) into the through guide groove 53e. The fitting between the needle support portion 52b and the through guide groove 53e is set relatively tight on condition that the sliding is not hindered such that the distal end of the arrowhead portion 52a4 of the needle-shaped member 52a is accurately guided to the through hole 51b of the other string support portion 51a. In other words, a relatively small clearance is set.

When the back side arm 52 is slid so as to further approach the front side arm 51, the needle support portion 52b is guided to the through guide groove 53e, the distal end of the arrowhead portion 52a4 of the needle-shaped member 52a is guided to the through hole 51b of the other string support portion 51a, and the needle support portion 52b abuts against the front end of the through guide groove 53e and stops. Performed as a result as illustrated in Figs. 7A and 7B is positioning at a predetermined position where the distal end of the arrowhead portion 52a4 of the needle-shaped member 52a can be appropriately engaged with the other engagement member 6b (see Fig. 1B) of the surgical string 6 supported in the accommodating space of the other string support portion 51a.

As described above, in the present embodiment, the projection portion 52e is guided to the recessed guide groove 53b and the needle support portion 52b is guided to the through guide groove 53e when the back side arm 52 is slid in the direction in which the back side arm 52 approaches the front side arm 51. As a result, the distal end of the arrowhead portion 52a4 of the needle-shaped member 52a is accurately guided to a predetermined position where the distal end can be appropriately engaged with the engagement members 6a and 6b of the surgical string 6 supported by the string support portion 51a. Accordingly, when the back side arm 52 is brought close to the front side arm 51, the shifting of the pivot-direction position of the back side arm 52 with respect to the front side arm 51 is effectively prevented. Accordingly, no re-alignment needs to be performed and a suture treatment can be quickly performed whereas the related art requires re-alignment in some cases.

In the present embodiment, guiding that is smooth as a whole can be performed since the relatively rough guide structure by means of the recessed guide grooves 53b and 53b and the projection portion 52e and the relatively precise guide structure by means of the through guide grooves 53e and 53e and the needle support portion 52b are combined. With only the relatively precise guide structure by means of the through guide grooves 53e and 53e and the needle support portion 52b, the clearance between the through guide grooves 53e and 53e and the needle support portion 52b is small, and thus the needle support portion 52b cannot be smoothly introduced into the through guide grooves 53e and 53e in some cases. However, it is possible to solve such a problem and realize guiding that is smooth as a whole by adding the relatively rough guide structure by means of the recessed guide grooves 53b and 53b and the projection portion 52e as in the present embodiment.

However, the relatively precise guide structure by means of the through guide grooves 53e and 53e and the needle support portion 52b may be used alone with the relatively rough guide structure by means of the recessed guide grooves 53b and 53b and the projection portion 52e omitted. In addition, the guide structure by means of the recessed guide grooves 53b and 53b and the projection portion 52e may be used alone with the guide structure by means of the through guide grooves 53e and 53e and the needle support portion 52b omitted. In this case, the clearance between the recessed guide grooves 53b and 53b and the projection portion 52e may be set relatively small. In the case of the single guide structure as compared with the case of the combination of both, structural simplification can be achieved although the single structure may be somewhat inferior from the viewpoint of smooth guiding.

### (Operation Unit)

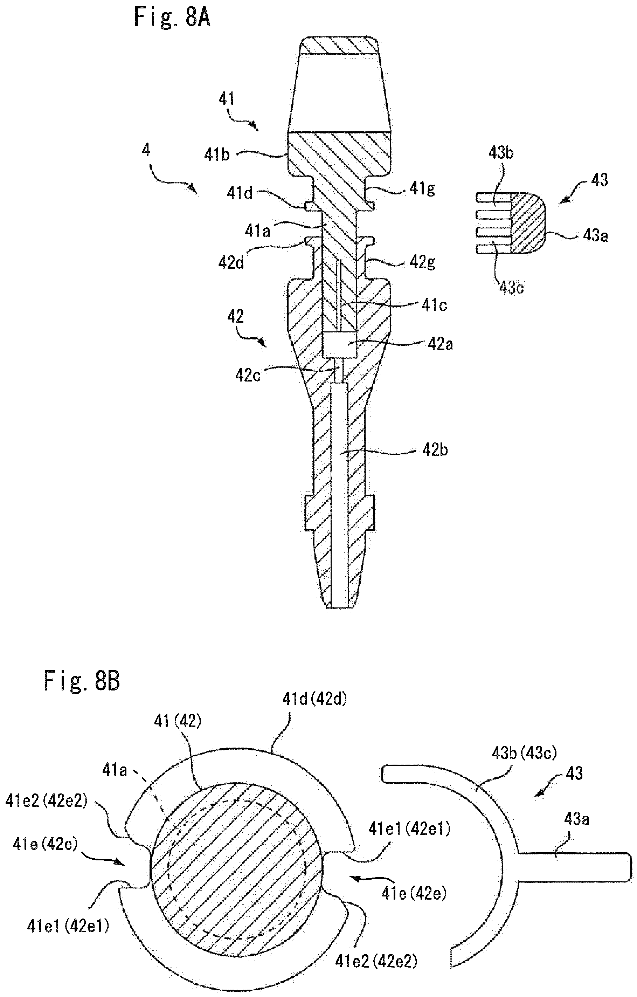

The operation unit (handle) 4 of the suturing device 1 is configured as illustrated in Figs. 1A, 8A, and 8B. In other words, the operation unit 4 is configured to be provided with a slider portion (first operation portion) 41, a base portion (second operation portion) 42, and a fixing holder (slide regulation means, fixing member) 43.

Schematically, the slider portion 41 is slidably provided on the base portion 42 and the slider portion 41 is slidable (movable) between a position where the slider portion 41 has moved to the distal end side with respect to the base portion 42 (pivot position) and a position where the slider portion 41 has moved to the proximal end side with respect to the base portion 42 (engagement position). When the slider portion 41 is at the pivot position with respect to the base portion 42, the front side arm 51 can be pivoted (turned) with respect to the back side arm 52. By sliding the slider portion 41 to the engagement position with respect to the base portion 42 after appropriately setting an angle in this state, it is possible to engage the arrowhead portion 52a4 of the needle-shaped member 52a with the engagement member 6a or 6b through the through hole 61a of the engagement member 6a or 6b of the surgical string 6 accommodated in one accommodating space 51f of the string support portions 51a and 51a of the front side arm 51.

The sliding of the slider portion 41 with respect to the base portion 42 is regulated, that is, both are fixed to each other by the fixing holder 43 being attached in a state where the position of the slider portion 41 with respect to the base portion 42 is at a predetermined position (halt position) between the pivot position and the engagement position.

As best illustrated in Fig. 8A, the distal end side of the slider portion 41 is a columnar portion 41a formed in a substantially columnar shape, the slider portion 41 has a grip portion 41b on the proximal end side of the slider portion 41, and the grip portion 41b has a distal end fixed to the columnar portion 41a. Formed in the columnar portion 41a of the slider portion 41 is a substantially columnar wire insertion hole 41c open to the distal end of the columnar portion 41a and extending along the axis of the columnar portion 41a.

The diameter of the wire insertion hole 41c is set to a value approximately equal to the outer diameter of the front side arm moving wire 31. The proximal end side of the front side arm moving wire 31 is inserted into the wire insertion hole 41c and the proximal end side part of the front side arm moving wire 31 is connected and fixed to the columnar portion 41a of the slider portion 41.

The base portion 42 is formed of a substantially cylindrical member formed by a pair of substantially symmetric half members being joined by a plurality of screws or the like (not illustrated) and has a substantially columnar slider portion insertion hole 42a open to the proximal end of the base portion 42 and extending along the axis of the base portion 42. The diameter of the slider portion insertion hole 42a is set to a value slightly larger than the outer diameter of the columnar portion 41a of the slider portion 41. The slider portion 41 is attached so as to be slidable along the axis with respect to the base portion 42 by the columnar portion 41a of the slider portion 41 being inserted into the slider portion insertion hole 42a. The longitudinal dimension (depth) of the slider portion insertion hole 42a (in the direction along the axis) is set to a value larger than the longitudinal dimension of the columnar portion 41a of the slider portion 41.

In addition, the base portion 42 has a substantially columnar tube insertion hole 42b open to the distal end of the base portion 42 and extending along the axis of the base portion 42. The tube insertion hole 42b is provided substantially coaxially with the slider portion insertion hole 42a. The diameter of the tube insertion hole 42b is set to a value approximately equal to the outer diameter of the back side arm moving tube 32. The proximal end side of the back side arm moving tube 32 is inserted into the tube insertion hole 42b and the proximal end side part of the back side arm moving tube 32 is connected and fixed to the base portion 42.

Further, the base portion 42 has a substantially columnar wire insertion hole 42c provided substantially coaxially with the slider portion insertion hole 42a and the tube insertion hole 42b such that communication is allowed between the slider portion insertion hole 42a and the tube insertion hole 42b. The diameter of the wire insertion hole 42c is set to a value slightly larger than the outer diameter of the front side arm moving wire 31. In a state where the proximal end side of the front side arm moving wire 31 drawn out from the proximal end of the back side arm moving tube 32 is slidably inserted through the wire insertion hole 42c, the proximal end side part of the front side arm moving wire 31 is connected and fixed to the columnar portion 41a.

In the present embodiment, a substantially disc ring-shaped flange portion (first projection portion) 41d is integrally provided at the distal end side part of the grip portion 41b of the slider portion 41 (part where the columnar portion 41a is provided). The flange portion 41d is a part constituting a part of the slide regulation means, is formed so as to be larger in diameter than the columnar portion 41a, and protrudes to the outside in the radial direction of the grip portion 41b. In addition, a substantially disc ring-shaped flange portion (second projection portion) 42d is integrally provided at the proximal end side part of the base portion 42, the flange portion 42d is a part constituting another part of the slide regulation means, and the flange portion 42d protrudes to the outside in the radial direction so as to face the flange portion 41d. By sliding (pushing) the slider portion 41 to the distal end side with respect to the base portion 42, it is possible to allow the distal end side surface of the flange portion 41d of the slider portion 41 to abut against the proximal end side surface of the flange portion 42d of the base portion 42.

In addition, as illustrated in Fig. 8B, a pair of notch portions 41e and 41e are formed at positions corresponding to each other at approximately 180° in the flange portion 41d of the slider portion 41. One inner surface 41e1 of the notch portion 41e is formed so as to be substantially parallel to a line passing through the center of the flange portion 41d and the other inner surface 41e2 is formed in a substantially circular arc shape and is smoothly connected to the outer peripheral surface of the flange portion 41d. In addition, likewise, a pair of notch portions 42e and 42e are formed at positions corresponding to each other at approximately 180° in the flange portion 42d of the base portion 42. One inner surface 42e1 of the notch portion 42e is formed so as to be substantially parallel to a line passing through the center of the flange portion 42d and the other inner surface 42e2 is formed in a substantially circular arc shape and is smoothly connected to the outer peripheral surface of the flange portion 42d. The notch portions 41e and 41e of the flange portion 41d and the notch portions 42e and 42e of the flange portion 42d are formed so as to correspond to each other. Although the number of the notch portions 41e of the flange portion 41d is two here and the number of the notch portions 42e of the flange portion 42d is two here, the numbers may be one or may be three or more. In a case where the numbers are three or more, the notch portions may be disposed at equiangular intervals.

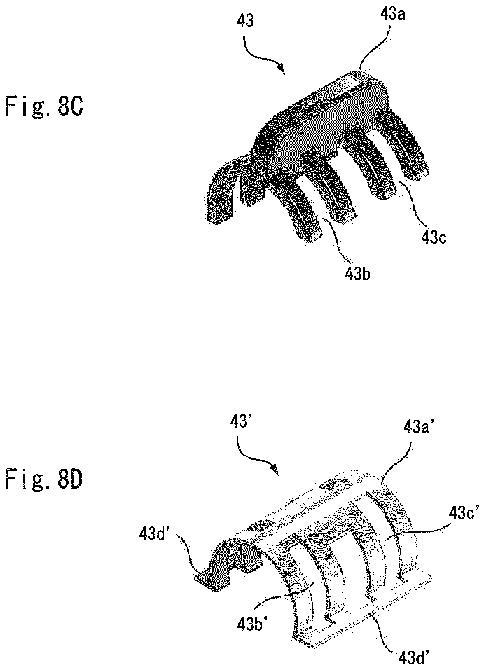

In addition, the operation unit 4 is provided with the fixing holder (fixing member) 43 as a member constituting a part of the rest of the slide regulation means. As illustrated in Fig. 8C, the fixing holder 43 has a grip portion (connecting portion) 43a for being gripped, attached, and detached by an operator's hand (finger) and two fitted portions (first and second fitted portions) 43b and 43c. Each of the fitted portions 43b and 43c is constituted by a pair of circular arc-shaped projection portions formed in a substantially circular arc shape. A part of the flange portion 41d of the slider portion 41 is fitted (inserted) into the part between the pair of circular arc-shaped projection portions of the fitted portion 43b and a part of the flange portion 42d of the base portion 42 is fitted (inserted) into the part between the pair of circular arc-shaped projection portions of the fitted portion 43c.

When the flange portion 41d and the flange portion 42d are fitted to the corresponding fitted portions 43b and 43c of the fixing holder 43, a part of the grip portion 43a on the circular arc-shaped projection portion side is inserted into the notch portion 41e of the flange portion 41d and the corresponding notch portion 42e of the flange portion 42d. The inner diameter of each circular arc-shaped projection portion that constitutes the fitted portion 43b is set substantially equal to the outer diameter of a neck part (neck portion) 41g of the grip portion 41b closer to the proximal end side than the flange portion 41d. The inner diameter of each circular arc-shaped projection portion that constitutes the fitted portion 43c is set substantially equal to the outer diameter of a neck part (neck portion) 42g of the base portion 42 closer to the distal end side than the flange portion 42d. It should be noted that the respective circular arc-shaped projection portions of the fitted portions 43b and 43c in the present embodiment have different lengths on one side and the other side across the grip portion 43a and are asymmetric. Although this is to facilitate attachment and detachment with respect to the slider portion 41 and the base portion 42, the respective circular arc-shaped projection portions of the fitted portions 43b and 43c may have the same length and be symmetric. The fixing holder 43 can be manufactured by resin mold molding or the like.

The dimension between the fitted portions 43b and 43c of the fixing holder 43 is set to a length at which the slider portion 41 and the base portion 42 can be fixed to each other in a state where the position of the slider portion 41 with respect to the base portion 42 is the halt position.

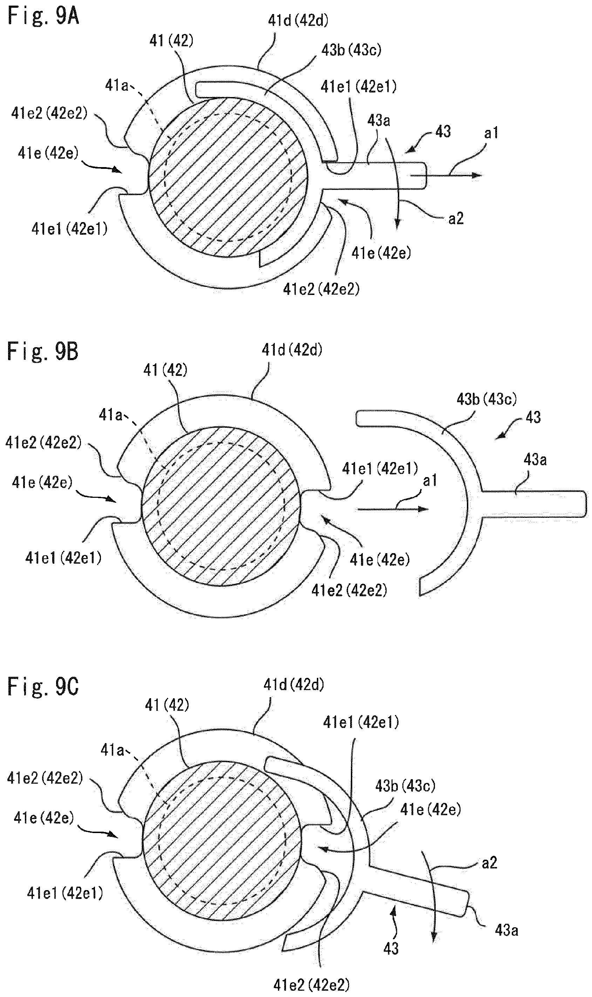

The slider portion 41 and the base portion 42 are fixed to each other by the fixing holder 43 such that the position of the slider portion 41 with respect to the base portion 42 becomes the halt position. In this state, the grip portion 43a of the fixing holder 43 is gripped as illustrated in Fig. 9A. Then, the grip portion 43a is pulled as indicated by the arrow a1 in Fig. 9A. As a result, it is possible to release the fitting of the flange portions 41d and 42d with respect to the corresponding fitted portions 43b and 43c of the fixing holder 43 and remove the fixing holder 43 as illustrated in Fig. 9B. In addition, in Fig. 9A, the grip portion 43a of the fixing holder 43 is gripped and pivoted as indicated by the arrow a2 in Fig. 9A, and then the part of the grip portion 43a on the circular arc-shaped projection portion side moves along the substantially circular arc-shaped side surfaces 41e2 (42e2) of the corresponding notch portions 41e and 42e of the flange portions 41d and 42d. As a result, it is possible to release the fitting of the flange portions 41d and 42d with respect to the corresponding fitted portions 43b and 43c of the fixing holder 43 and remove the fixing holder 43 as illustrated in Fig. 9C. An operator can select any of these methods to remove the fixing holder 43. It should be noted that two notch portions 41e of the flange portion 41d and two notch portions 42e of the flange portion 42d in the present embodiment are disposed at positions corresponding at approximately 180° and one of these is selected and used as appropriate when the fixing holder 43 is attached.

The fixing holder that fixes the slider portion 41 and the base portion 42 to each other is not limited to the fixing holder having the configuration of the fixing holder 43 illustrated in Fig. 8C. For example, a fixing holder 43' having the configuration illustrated in Fig. 8D may also be used. The fixing holder 43' is configured to be provided with a middle connecting portion (connecting portion) 43a' connecting the pair of circular arc-shaped projection portions that respectively constitute fitted portions 43b' and 43c' to each other and provided with distal end connecting portions 43d' and 43d' connecting the distal end portions of the circular arc-shaped projection portions to each other. The fixing holder 43' can be manufactured by a sheet metal being punched and pressed.

Although the respective circular arc-shaped projection portions of the fitted portions 43b' and 43c' of the fixing holder 43' have different lengths on one side and the other side across the middle connecting portion 43a' and are symmetric, the respective circular arc-shaped projection portions of the fitted portions 43b' and 43c' of the fixing holder 43' may have different lengths and be asymmetric as in the case of the fixing holder 43. In addition, although the distal end connecting portion 43d' is provided for each circular arc-shaped projection portion to be reinforced, the distal end connecting portion 43d' may not be provided in a case where strength can be sufficiently ensured. Further, the middle connecting portion 43a' may have a shape that allows an operator to grip the middle connecting portion 43a' with his or her hand (finger).

As illustrated in Figs. 1B and 4A, in a state where the position of the slider portion 41 with respect to the base portion 42 is at the predetermined halt position between the pivot position and the engagement position, a halted state occurs where the arrowhead portion 52a4 of the needle-shaped member 52a has approached, without engagement, one of the engagement members 6a and 6b accommodated in the accommodating space 51f in the suturing unit 5. It is possible to maintain the halted state by attaching the fixing holder 43 such that the position of the slider portion 41 with respect to the base portion 42 becomes the halt position.

The "halted state" is a state where the distal end of the needle-shaped member 52a (arrowhead portion 52a4) is within the range of the following halt front end position to the following halt rear end position. Here, the halt front end position is the position that is immediately ahead of the engagement between the needle-shaped member 52a and the engagement members 6a and 6b (immediately ahead of the passage of the rear end (maximum-diameter part) of the arrowhead portion 52a4 through the through hole 61a of the engagement members 6a and 6b) although the distal end (minimum-diameter part) of the tapered needle-shaped member 52a (arrowhead portion 52a4) is in one through hole 61a of the engagement members 6a and 6b accommodated in the accommodating space 51f. In addition, the halt rear end position is a position at which a body tissue constituting the inner wall of a body lumen does not enter between the rear surface of the string support portion 51a and the distal end of the needle-shaped member 52a (arrowhead portion 52a4) when the suturing unit 5 advances in the body lumen although the distal end of the needle-shaped member 52a (arrowhead portion 52a4) is separated to the proximal end side from the rear surface (proximal end side surface) of the string support portion 51a. Specifically, the halt rear end position is a position where the distance (dimension d illustrated in Fig. 4B) between the distal end of the needle-shaped member 52a (arrowhead portion 52a4) and the rear surface of the string support portion 51a (surface of the engagement member fixing film 51g) is 3 mm or less and more preferably a position where the distance is 1 mm or less.

Here, even in a state where the fixing holder 43 is attached in the operation unit 4 and the slider portion 41 and the base portion 42 are fixed to each other, the sheath unit 3 is curved or the like by following the posture of the endoscope 2 to which the suturing device 1 is attached and the posture of the sheath unit 3 changes when the suturing device 1 is inserted into the body. Fluctuations in relative position result from the difference in curvature between the front side arm moving wire 31 and the back side arm moving tube 32 constituting the sheath unit 3, and thus the positional relationship of the distal end of the needle-shaped member 52a (arrowhead portion 52a4) with respect to the string support portion 51a fluctuates in the suturing unit 5. Accordingly, it is desirable to set the above-described halt position such that the distal end of the needle-shaped member 52a (arrowhead portion 52a4) is within the above-described range of the halt front end position to the halt rear end position even in the event of fluctuations in the posture of the sheath unit 3 that may arise during the use of the suturing device 1.

In the present embodiment, the flange portions 41d and 42d and the fixing holder 43 are provided as the slide regulation means for releasably regulating the sliding of the slider portion 41 and the base portion 42 in a state where the relative positions of the slider portion 41 and the base portion 42 are adjusted for the halted state in the suturing unit 5, that is, at the halt position. In the halted state, the distal end of the arrowhead portion 52a4 of the needle-shaped member 52a is in the accommodating space 51f of the string support portion 51a of the front side arm 51 or at a position close to the rear surface (proximal end side surface) of the string support portion 51a. Accordingly, when the suturing unit 5 is inserted into the body lumen, the string support portion 51a functions as a protective member that protects the distal end of the needle-shaped member 52a (arrowhead portion 52a4), and thus it is possible to prevent the distal end from interfering with the inner wall of the body lumen or another part. Accordingly, the insertion into the body can be smoothly carried out.

In addition, such a function is realized only in the operation unit 4 without any change in the configuration in the suturing unit 5. Accordingly, the enlarged part of the needle accommodating arm or the string support portion does not act as a hindrance and obstructs no treatment as compared with the structure according to the related art in which the third arm (needle accommodating arm) having the needle accommodating hole is provided at the part between the pair of string support portions of the front side arm or the width of one string support portion is increased for the needle accommodating hole to be formed adjacent to the accommodating portion accommodating the engagement member of the surgical string and the needle accommodating hole to accommodate the distal end portion of the needle-shaped member.

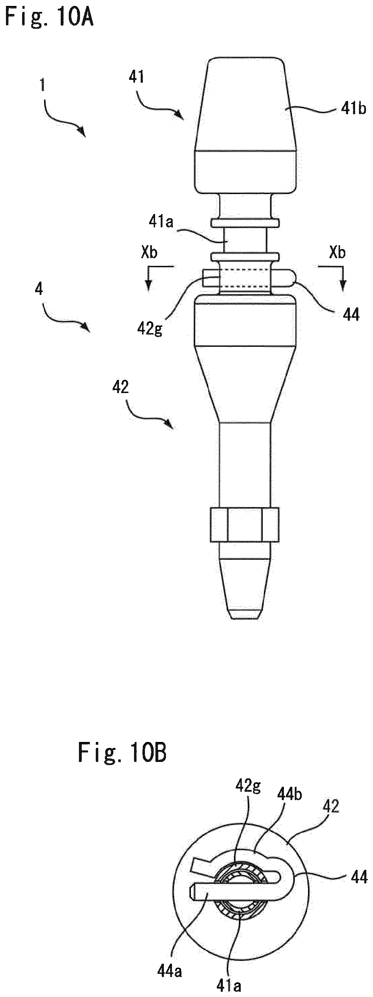

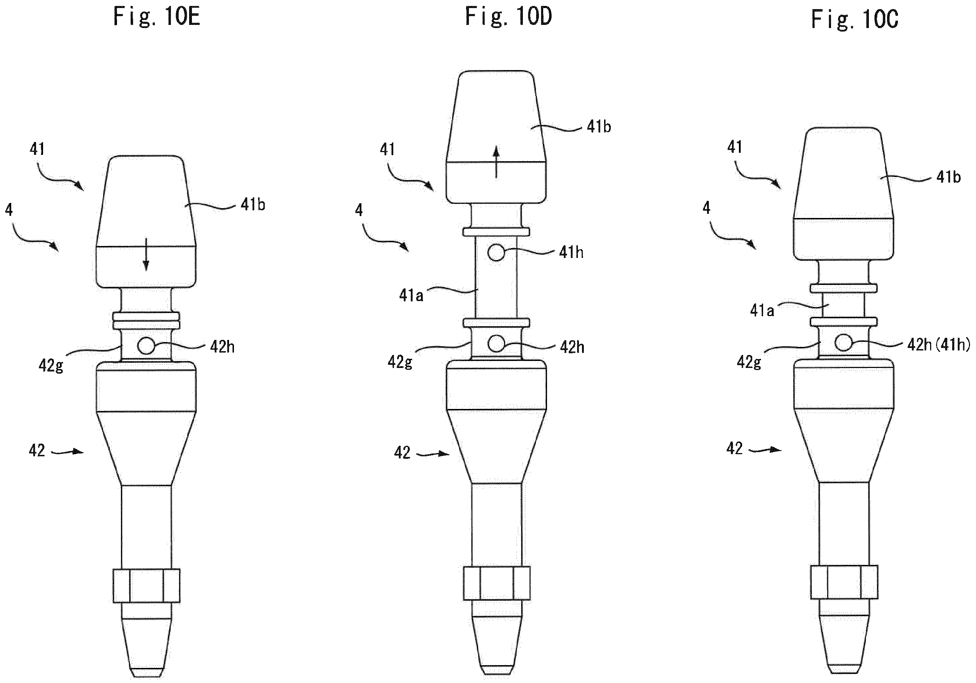

The slide regulation means for releasably regulating the sliding of the slider portion 41 and the base portion 42 is not limited to the above and one having another configuration may also be used. For example, what is illustrated in Figs. 10A to 10E may be used. In other words, a through hole (first through hole portion) 41h is formed in the columnar portion 41a of the slider portion 41 in a direction substantially orthogonal to the axial direction of the columnar portion 41a and a through hole (second through hole portion) 42h is similarly formed at the neck part (neck portion) 42g on the proximal end side of the base portion 42 (see Fig. 10D). The through holes 41h and 42h are formed so as to become mutually corresponding positions in a state where the relative positions of the slider portion 41 and the base portion 42 are adjusted for the above-described halted state (see Fig. 10C).

Then, in this state, a straight portion 44a of a pin member 44 formed in a substantially hairpin shape and having the straight portion 44a and a curved portion 44b is inserted into the through holes 41h and 42h. As a result, a part (approximately half) of the outer periphery of the neck portion 42g is sandwiched between the straight portion 44a and the curved portion 44b and the pin member 44 is attached to the neck portion 42g of the base portion 42 so as to be detachable (insertable and removable).

When the suturing unit 5 is inserted into the body lumen, this is performed in a state where the pin member 44 is attached and the slider portion 41 and the base portion 42 are fixed to each other with the sliding of the slider portion 41 with respect to the base portion 42 regulated. As illustrated in Fig. 10C, when the suturing unit 5 has reached the vicinity of the part to be sutured in the body lumen, the regulation is released by the pin member 44 being pulled out from the through holes 41h and 42h. Then, sliding becomes possible between the engagement position illustrated in Fig. 10D and the pivot position illustrated in Fig. 10E. The other configurations and effects are the same as those of the above-described embodiment.

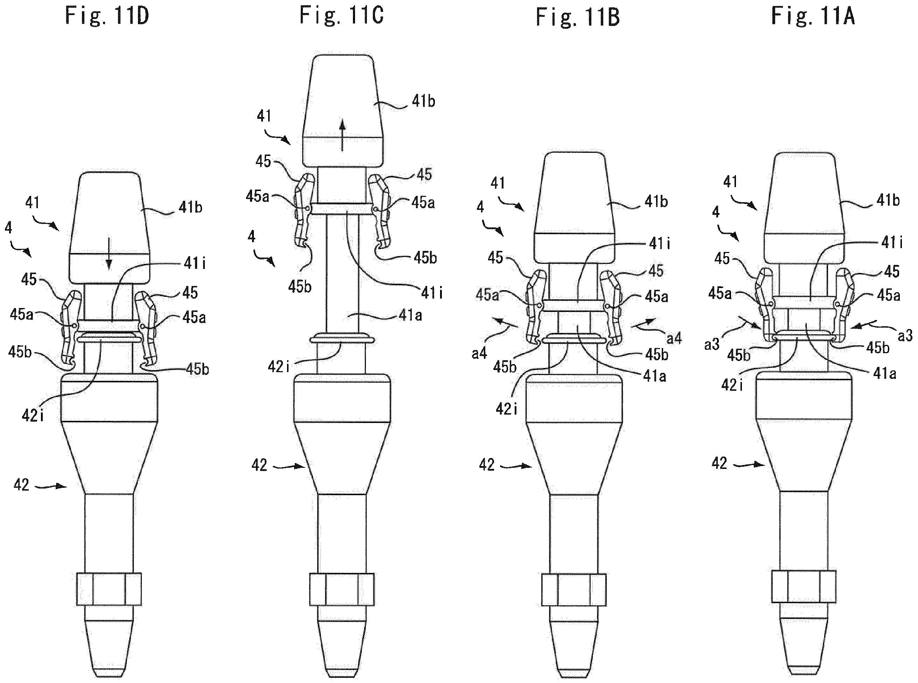

In addition, one having yet another configuration may be used as the slide regulation means. For example, what is illustrated in Figs. 11A to 11D may be used. In other words, a substantially ring-shaped flange portion (engaged portion) 42i overhanging in a flange shape is provided at the proximal end side part of the base portion 42 and a pair of clip members 45 and 45 are disposed substantially symmetrically at positions corresponding to each other at approximately 180° in a flange portion 41i provided on the distal end side of the grip portion 41b. The clip members 45 and 45 are respectively and pivotably supported by a pair of pivot shafts 45a and 45a having intermediate parts substantially orthogonal to the axial direction of the slider portion 41 (direction in which the slider portion 41 slides). The clip members 45 and 45 are configured to pivot when intentionally pressed, not to pivot unless a certain amount of force is applied, and to maintain the posture at that time. Formed inside the distal end portions of the clip members 45 and 45 are engagement portions 45b and 45b shaped so as to releasably engage with a part of the outer peripheral part of the flange portion 42i.

As illustrated in Fig. 11A, with the relative positions of the slider portion 41 and the base portion 42 adjusted for the halted state, the parts of the pair of clip members 45 and 45 closer to the distal end side than the respective pivot shafts 45a and 45a are pressed so as to approach each other (as indicated by the arrows a3 and a3 in Fig. 11A). As a result, the engagement portions 45b and 45b in the distal end portions of the pair of clip members 45 and 45 are respectively engaged with a part of the flange portion 42i of the base portion 42 and the sliding of the slider portion 41 with respect to the base portion 42 can be regulated.

When the suturing unit 5 is inserted into the body lumen, this is performed with the sliding of the slider portion 41 with respect to the base portion 42 regulated. When the suturing unit 5 has reached the vicinity of the part to be sutured in the body lumen, the parts of the pair of clip members 45 and 45 closer to the proximal end side than the respective pivot shafts 45a and 45a are pressed so as to approach each other as illustrated in Fig. 11B. As a result, the distal end portions of the pair of clip members 45 and 45 are opened as indicated by the arrows a4 and a4 in Fig. 11B and each engagement with a part of the flange portion 42i of the base portion 42 is released. As a result, the regulation of the sliding of the slider portion 41 with respect to the base portion 42 is released and sliding becomes possible between the engagement position illustrated in Fig. 11C and the pivot position illustrated in Fig. 11D. The other configurations and effects are the same as those of the above-described embodiment.

It should be noted that the slider portion 41 may be provided with a flange portion and the base portion 42 may be provided with a pair of clip members on the contrary to the base portion 42 being provided with the flange portion 42i and the slider portion 41 being provided with the pair of clip members 45 and 45 here. In addition, the clip member may be provided alone.

### (Suturing Process)

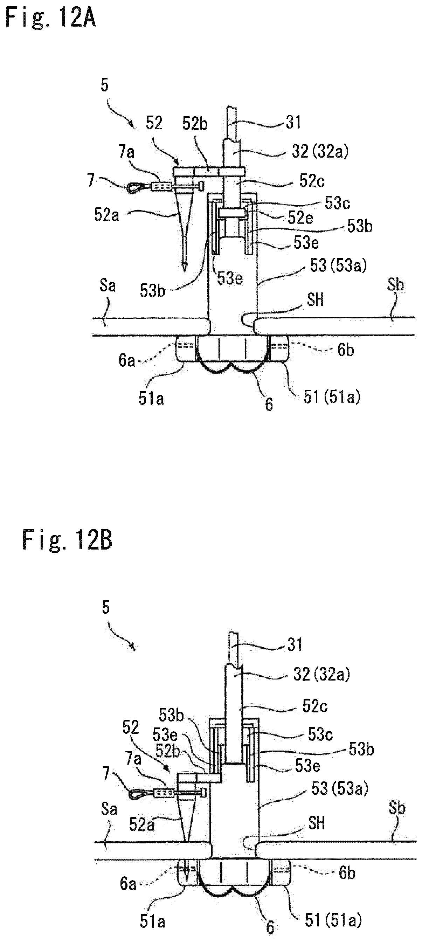

Hereinafter, a suturing process (suture operation) for an incision by means of the suturing device 1 described above will be described with reference to Figs. 12A to 12K. It should be noted that a case where an incision formed in a stomach wall is sutured will be described as an example below.

First, the fixing holder 43 is attached in the operation unit 4 as indicated by a dotted line in Fig. 1A and the suturing device 1 in the halted state is attached to the shaft of the endoscope 2 in the suturing unit 5 as illustrated in Fig. 1B. Then, these are inserted into the stomach and the suturing unit 5 of the suturing device 1 is disposed in the vicinity of the incision to be sutured. Next, the fixing holder 43 is removed in the operation unit 4, and then the slider portion 41 is slid in the direction of pushing to the distal end side with respect to the base portion 42 and the back side arm 52 is separated from the front side arm 51.

In this state, the first process that is illustrated in Fig. 12A is performed. In the first process illustrated in Fig. 12A, the operation unit 4 is pushed first into the distal end side as a whole with respect to the case tube 33 and rotated as necessary, which results in only the front side arm 51 that is spaced apart from the back side arm 52 being inserted into an incision SH as illustrated in Fig. 12A. Before and after this, the back side arm 52 is pivoted with respect to the front side arm 51 and the front side arm 51 and the back side arm 52 are disposed such that one edge portion Sa of the incision SH is sandwiched between one string support portion 51a of the front side arm 51 and the needle-shaped member 52a (arrowhead portion 52a4).

Next, the slider portion 41 is slid in the direction of pulling to the proximal end side with respect to the base portion 42 in the operation unit 4 and the front side arm 51 is brought close to the back side arm 52 in the suturing unit 5. Then, as illustrated in Fig. 12B, the needle-shaped member 52a pierces one edge portion Sa and penetrates the edge portion Sa and the arrowhead portion 52a4 of the needle-shaped member 52a passes through the through hole of the engagement member 6a accommodated (supported) in the accommodating space of one string support portion 51a. As a result, the through hole of the engagement member 6a reaches the small-diameter portion 52a3 of the needle-shaped member 52a and the engagement member 6a is engaged with the needle-shaped member 52a.

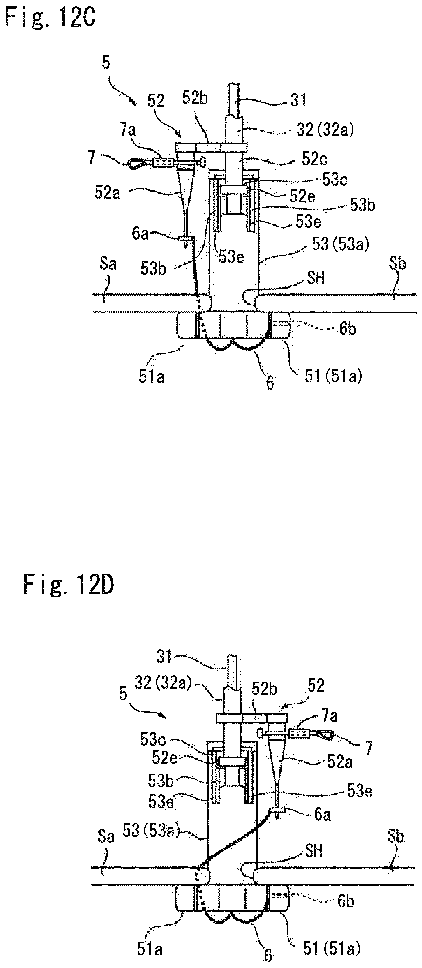

Next, the front side arm 51 is separated from the back side arm 52 in the suturing unit 5 by the operation unit 4 being operated. Then, as illustrated in Fig. 12C, the needle-shaped member 52a with which the engagement member 6a is engaged returns to the inside of the stomach by moving backward through the hole (hereinafter, referred to as a first perforation) formed during the insertion through one edge portion Sa. As a result, a part of the surgical string 6 (part on the engagement member 6a side) penetrates one edge portion Sa.

Then, as illustrated in Fig. 12D, the front side arm 51 and the back side arm 52 are disposed such that the other edge portion Sb of the incision SH is sandwiched between the needle-shaped member 52a and the other string support portion 51a of the front side arm 51.

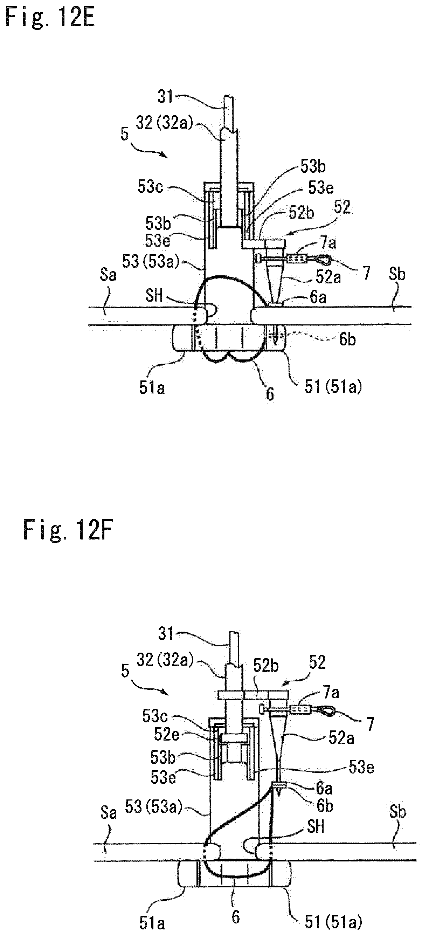

In this state, the back side arm 52 is brought close to the front side arm 51 by the operation unit 4 being operated. Then, as illustrated in Fig. 12E, the needle-shaped member 52a pierces the other edge portion Sb and penetrates the edge portion Sb and the arrowhead portion 52a4 of the needle-shaped member 52a passes through the through hole of the engagement member 6b accommodated (supported) in the accommodating space of the other string support portion 51a. As a result, the through hole of the engagement member 6b reaches the small-diameter portion 52a3 of the needle-shaped member 52a and the engagement member 6b is engaged with the needle-shaped member 52a.

Next, the front side arm 51 is separated from the back side arm 52 by the operation unit 4 being operated. Then, as illustrated in Fig. 12F, the needle-shaped member 52a with which the engagement member 6b is engaged returns to the inside of the stomach by moving backward through the hole (hereinafter, referred to as a second perforation) formed during the insertion through the other edge portion Sb. As a result, a part of the surgical string 6 (part on the engagement member 6b side) penetrates the other edge portion Sb.

As a result, each of the pair of engagement members 6a and 6b to which both ends of the surgical string 6 are fixed is engaged with the single needle-shaped member 52a and the surgical string 6 forms a ring that reaches the outside of the stomach through the first perforation from the needle-shaped member 52a (that is, the inside of the stomach) and returns to the needle-shaped member 52a (that is, the inside of the stomach) through the second perforation from the outer surface of the stomach.

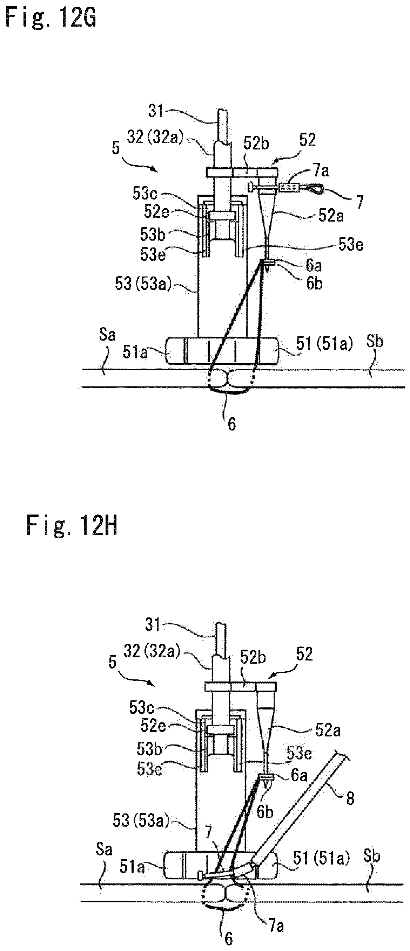

Next, the front side arm 51 (string support portion 51a) is moved into the stomach through the incision SH as illustrated in Fig. 12G by the operation unit 4 being operated. With the front side arm 51 (string support portion 51a) in the stomach, the needle-shaped member 52a is separated from the incision SH and both ends of the surgical string 6 move away from the incision SH. As a result, the part of the surgical string 6 penetrating the first perforation and the part of the surgical string 6 penetrating the second perforation are drawn together and the end surfaces of the pair of edge portions Sa and Sb of the incision SH are joined by abutting against each other.

The ligation of the surgical string 6 is performed after the suturing of the incision SH by means of the suturing device 1 is completed. A ligation device (not illustrated) is used for the ligation. Usable as the ligation device is, for example, a ligation device provided with a ligation tube introduced into a lumen via a treatment tool guide tube of an endoscope and a ligation wire slidably inserted through the ligation tube and having a configuration in which the distal end of the ligation wire is provided with a connecting hook opened in a substantially V shape by the elasticity of the connecting hook by protruding from the distal end of the tube and closed by being drawn into the distal end portion of the tube.

As illustrated in Fig. 12G, the ligation tool 7 is held in a state where the ligation loop 7c (see Fig. 1B) is inserted in the proximal end portion (large-diameter portion 52a1) of the needle-shaped member 52a. Accordingly, the ligation tool 7 is connected to the distal end of a ligation tube 8 (see Fig. 12H) by the distal end of the ligation tube 8 of the ligation device being disposed in the vicinity of the connecting loop 7b of the ligation tool 7, the connecting loop 7b being gripped by means of the connecting hook open by protruding from the ligation tube 8, and the connecting loop 7b being drawn into the distal end portion of the ligation tube 8.

Next, as illustrated in Fig. 12H, the distal end portion of the ligation tube 8 to which the ligation tool 7 is connected is moved to the incision SH side (downward in Fig. 12H) and both end portions of the surgical string 6 are inserted through the ligation loop 7c of the ligation tool 7. As a result, the surgical string 6 is squeezed and both end portions of the surgical string 6 are bundled.

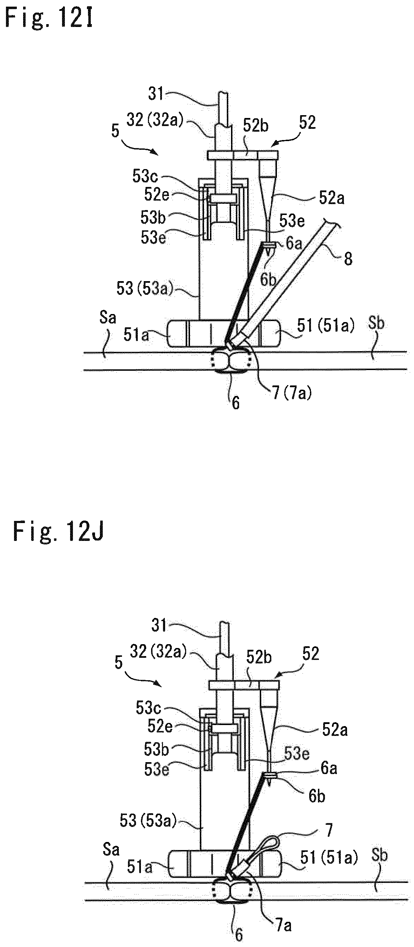

With both end portions of the surgical string 6 bundled, the ligature wire is slid such that the connecting hook is further drawn into the ligation tube 8. Then, as illustrated in Fig. 12I, the ligation loop 7c is drawn into the tightening tube 7a together with the surgical string 6 and the ligation loop 7c and the surgical string 6 are accommodated in the tightening tube 7a in a state of being in close contact with each other and compressed. In other words, the ligation loop 7c and the surgical string 6 are accommodated in a state of being tightly fitted in the tube 7a, and thus one end portion of the surgical string 6 and one end portion of the ligation loop 7c are fixed so as not to fall from the tube 7a. As a result, the ligation of the surgical string 6 is completed.

After the ligation of the surgical string 6 is completed, the ligation tube 8 is slid to the proximal end side so as to be drawn with respect to the ligation wire with the position of the ligation wire unchanged. Then, the other end portion of the loop member and the connecting hook protrude (are exposed) from the distal end of the ligation tube 8, the connecting hook is opened by the elasticity of the connecting hook, the grip is released, and the ligation device is separated from the ligated ligation tool 7 as illustrated in Fig. 12J.

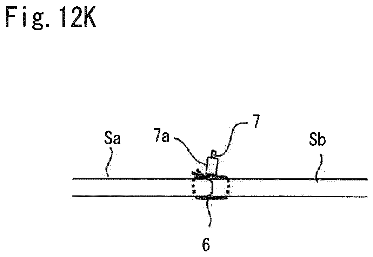

Lastly, as illustrated in Fig. 12K, the ligation tool 7 and the suturing unit 5 are separated by the surgical string 6 between the ligation tool 7 and the suturing unit 5 (needle-shaped member 52a) being cut with a pair of endoscopic scissors forceps called a loop cutter or the like. Further, if necessary, a series of procedures related to singleneedle suturing is completed by the other end portion of the loop member of the ligated ligation tool 7 being cut by means of the loop cutter or the like and collected.

The embodiment described above has been described so that the invention is understood with ease and does not limit the invention. Accordingly, each element disclosed in the embodiment described above is intended to include every design change and equivalent belonging to the technical scope of the invention.

### EXPLANATIONS OF LETTERS OR NUMERALS

1 SUTURING DEVICE
2 ENDOSCOPE
3 SHEATH UNIT
   31 FRONT SIDE ARM MOVING WIRE (FIRST LONG MEMBER)
   32 BACK SIDE ARM MOVING TUBE (SECOND LONG MEMBER)
   33 CASE TUBE
4 OPERATION UNIT
41 SLIDER PORTION (FIRST OPERATION PORTION)
   41a COLUMNAR PORTION
   41b GRIP PORTION
   41d FLANGE PORTION (SLIDE REGULATION MEANS, FIRST PROJECTION PORTION)
   41e NOTCH PORTION
   41g NECK PORTION
   41h THROUGH HOLE (SLIDE REGULATION MEANS, FIRST THROUGH HOLE PORTION)
   41i FLANGE PORTION
42 BASE PORTION (SECOND OPERATION PORTION)
   42d FLANGE PORTION (SLIDE REGULATION MEANS, SECOND PROJECTION PORTION)
   42e NOTCH PORTION
   42g NECK PORTION
   42h THROUGH HOLE (SLIDE REGULATION MEANS, SECOND THROUGH HOLE PORTION)
   42i FLANGE PORTION (SLIDE REGULATION MEANS, ENGAGED PORTION)
   43, 43' FIXING HOLDER (SLIDE REGULATION MEANS, FIXING MEMBER)
   43a GRIP PORTION (CONNECTING PORTION)
   43b, 43c FITTED PORTION (FIRST FITTED PORTION, SECOND FITTED PORTION)
   44 PIN MEMBER (SLIDE REGULATION MEANS)
   45 CLIP MEMBER (SLIDE REGULATION MEANS)
   45a PIVOT SHAFT
   45b ENGAGEMENT PORTION
5 SUTURING UNIT
   51 FRONT SIDE ARM (FIRST ARM)
      51a STRING SUPPORT PORTION
      51b THROUGH HOLE
      51f ACCOMMODATING SPACE (ENGAGEMENT MEMBER ACCOMMODATING PORTION)
      51g ENGAGEMENT MEMBER FIXING FILM
   52 BACK SIDE ARM (SECOND ARM)
      52a NEEDLE-SHAPED MEMBER
      52a4 ARROWHEAD PORTION (DISTAL END PORTION OF NEEDLE-SHAPED MEMBER)
      52b NEEDLE SUPPORT PORTION
      52c BACK SIDE GUIDE PORTION
      52d PROJECTION MEMBER
      52e PROJECTION PORTION
   53 FRONT SIDE GUIDE PORTION
      53a THICK PORTION
      53b RECESSED GUIDE GROOVE
      53c CONNECTION GROOVE
      53d BOTTOM SIDE PORTION
      53e THROUGH GUIDE GROOVE
      53f INCLINED PORTION
      53g CHAMFERED PORTION
   54 CAP
6 SURGICAL STRING
   6a, 6b ENGAGEMENT MEMBER
   61a THROUGH HOLE
7 LIGATION TOOL
   7a TIGHTENING TUBE
   7b CONNECTING LOOP
   7c LIGATION LOOP
SH INCISION
Sa, Sb EDGE PORTION
8 LIGATION TUBE (LIGATION DEVICE)

## Claims

1. A suturing device (1) comprising:
a sheath unit (3) having a first long member (31) and a second long member (32) each having a distal end and a proximal end, the second long member (32) being provided so as to be slidable in an axial direction with respect to the first long member (31);
a suturing unit (5) provided at a distal end of the sheath unit (3); and
an operation unit (4) provided at a proximal end of the sheath unit (3), wherein
the suturing unit (5) comprises
a first arm (51) connected to the distal end of the first long member (31) and having a string support portion (51a) provided with an engagement member accommodating portion (51f) configured for releasably accommodating an engagement member (6a, 6b) provided at an end portion of a surgical string, and
a second arm (52) having a needle-shaped member (52a), provided so as to be slidable with respect to the first arm (51), and connected to the distal end of the second long member (32), and
the operation unit (4) comprises
a first operation portion (41) connected to the proximal end of the first long member (31), and
a second operation portion (42) connected to the proximal end of the second long member (32) and provided so as to be slidable with respect to the first operation portion (41), **characterized in that**
the operation unit (4) further comprises slide regulation means (41d, 42d, 43, 43')(41h, 42h, 44)(45, 42i) for releasably fixing the first operation portion (41) and the second operation portion (42) in a state where the first operation portion (41) and the second operation portion (42) are disposed such that a halted state occurs where a distal end portion (52a4) of the needle-shaped member (52a) is in the engagement member accommodating portion (51f) or at a position where the distance from a surface at the proximal end of the string support portion (51a) is 3 mm or less without engagement with the engagement member (6a, 6b).

2. The suturing device (1) according to claim 1, wherein
the slide regulation means (41d, 42d, 43, 43') comprises a first projection portion (41d) provided on the first operation portion (41), a second projection portion (42d) provided on the second operation portion (42) so as to correspond to the first projection portion (41d), and a fixing member (43, 43'), and
the fixing member (43, 43') comprises a first fitted portion (43b, 43b') having one end portion into which a part of the first projection portion (41d) can be detachably fitted in the halted state, a second fitted portion (43c, 43c') having the other end portion into which a part of the second projection portion (42d) can be detachably fitted in the halted state, and a connecting portion (43a, 43a') integrally connecting the first fitted portion (43b, 43b') and the second fitted portion (43c, 43c').

3. The suturing device (1) according to claim 1, wherein
the slide regulation means (41h, 42h, 44) comprises a first through hole portion (41h) provided in the first operation portion (41), a second through hole portion (42h) provided in the second operation portion (42) so as to correspond to the first through hole portion (41h) in the halted state, and a pin member (44) insertable in an insertable and removable manner into the first through hole portion (41h) and the second through hole portion in a state where the first through hole portion and the second through hole portion (42h) correspond to each other.

4. The suturing device (1) according to claim 1, wherein
the slide regulation means (45, 42i) comprises a clip member (45) pivotably provided on one of the first operation portion (41) and the second operation portion (42) and an engaged portion (42i) provided in the other of the first operation portion (41) and the second operation portion (42), a distal end portion (45b) of the clip member (45) releasably engageable with the engaged portion (42i), and
the distal end portion (45b) of the clip member (45) can engage with the engaged portion (42i) by the clip member (45) being pivoted in a predetermined direction in the halted state and the engagement of the distal end portion (45b) of the clip member (45) with the engaged portion (42i) can be released by the clip member (45) being pivoted in a direction opposite to the predetermined direction.

## Patentansprüche

1. Eine Nahtvorrichtung (1), umfassend:
eine Hülleneinheit (3) umfassend ein erstes langes Element (31) und ein zweites langes Element (32), die jeweils ein distales Ende und ein proximales Ende aufweisen, wobei das zweite lange Element (32) so vorgesehen ist, dass es in einer axialen Richtung in Bezug auf das erste lange Element (31) verschiebbar ist;
eine Näheinheit (5), die an einem distalen Ende der Hülseneinheit (3) vorgesehen ist; und
eine Betätigungseinheit (4), die an einem proximalen Ende der Hülleneinheit (3) vorgesehen ist, wobei
die Näheinheit (5) umfasst
einen ersten Arm (51), der mit dem distalen Ende des ersten langen Elements (31) verbunden ist und einen Schnurstützabschnitt (51a) aufweist, der mit einem Eingriffselement-Aufnahmeabschnitt (51f) versehen ist, der zum lösbaren Aufnehmen eines Eingriffselements (6a, 6b) eingerichtet ist, das an einem Endabschnitt einer chirurgischen Schnur vorgesehen ist, und
einen zweiten Arm (52) mit einem nadelförmigen Element (52a), das so vorgesehen ist, dass es in Bezug auf den ersten Arm (51) verschiebbar ist, und das mit dem distalen Ende des zweiten langen Elements (32) verbunden ist, und
die Betätigungseinheit (4) umfasst
einen ersten Betätigungsabschnitt (41), der mit dem proximalen Ende des ersten langen Elements (31) verbunden ist, und
einen zweiten Betätigungsabschnitt (42), der mit dem proximalen Ende des zweiten langen Elements (32) verbunden und so vorgesehen ist, dass er in Bezug auf den ersten Betätigungsabschnitt (41) verschiebbar ist, **dadurch gekennzeichnet, dass**
die Betätigungseinheit (4) ferner ein Schieberegelungsmittel (41d, 42d, 43, 43') (41h, 42h, 44) (45, 42i) zum lösbaren Fixieren des ersten Betätigungsabschnitts (41) und des zweiten Betätigungsabschnitts (42) in einem Zustand, in dem der erste Betätigungsabschnitt (41) und der zweite Betätigungsabschnitt (42) so angeordnet sind, dass ein angehaltener Zustand auftritt, in dem sich ein distaler Endabschnitt (52a4) des nadelförmigen Elements (52a) in dem Eingriffselement-Aufnahmeabschnitt (51f) oder in einer Position befindet, in der der Abstand von einer Oberfläche am proximalen Ende des Schnurstützabschnitts (51a) 3 mm oder weniger beträgt, ohne dass ein Eingriff mit dem Eingriffselement (6a, 6b) vorliegt.

2. Die Nahtvorrichtung (1) nach Anspruch 1, wobei
das Schieberegulierungsmittel (41d, 42d, 43, 43') einen ersten Vorsprung (41d), der an dem ersten Betätigungsabschnitt (41) vorgesehen ist, einen zweiten Vorsprung (42d), der an dem zweiten Betätigungsabschnitt (42) vorgesehen ist, so dass er dem ersten Vorsprung (41d) entspricht, und ein Befestigungselement (43, 43') umfasst, und
das Befestigungselement (43, 43') einen ersten eingepassten Abschnitt (43b, 43b') mit einem Endabschnitt, in den ein Teil des ersten Vorsprungsabschnitts (41d) in dem angehaltenen Zustand lösbar eingepasst werden kann, einen zweiten eingepassten Abschnitt (43c, 43c') mit dem anderen Endabschnitt, in den ein Teil des zweiten Vorsprungabschnitts (42d) im angehaltenen Zustand lösbar eingepasst werden kann, und einen Verbindungsabschnitt (43a, 43a'), der den ersten eingepassten Abschnitt (43b, 43b') und den zweiten eingepassten Abschnitt (43c, 43c') einstückig verbindet, umfasst.

3. Die Nahtvorrichtung (1) nach Anspruch 1, wobei
das Schieberegulierungsmittel (41h, 42h, 44) einen ersten Durchgangslochabschnitt (41h), der in dem ersten Betätigungsabschnitt (41) vorgesehen ist, einen zweiten Durchgangslochabschnitt (42h), der in dem zweiten Betätigungsabschnitt (42) vorgesehen ist, so dass er dem ersten Durchgangslochabschnitt (41h) in dem angehaltenen Zustand entspricht, und ein Stiftelement (44) umfasst, das in einer einführbaren und entfernbaren Weise in den ersten Durchgangslochabschnitt (41h) und den zweiten Durchgangslochabschnitt in einem Zustand einführbar ist, in dem der erste Durchgangslochabschnitt und der zweite Durchgangslochabschnitt (42h) einander entsprechen.

4. Die Nahtvorrichtung (1) nach Anspruch 1, wobei
das Schieberegulierungsmittel (45, 42i) ein Klammerelement (45), das schwenkbar an einem von dem ersten Betätigungsabschnitt (41) und dem zweiten Betätigungsabschnitt (42) vorgesehen ist, und einen Eingriffsabschnitt (42i), der in dem anderen von dem ersten Betätigungsabschnitt (41) und dem zweiten Betätigungsabschnitt (42) vorgesehen ist, umfasst, wobei ein distaler Endabschnitt (45b) des Klammerelements (45) lösbar mit dem Eingriffsabschnitt (42i) in Eingriff gebracht werden kann, und
der distale Endabschnitt (45b) des Klammerelements (45) mit dem Eingriffsabschnitt (42i) in Eingriff gebracht werden kann, indem das Klammerelement (45) im angehaltenen Zustand in eine vorbestimmte Richtung geschwenkt wird, und der Eingriff des distalen Endabschnitts (45b) des Klammerelements (45) mit dem Eingriffsabschnitt (42i) gelöst werden kann, indem das Klammerelement (45) in eine Richtung entgegengesetzt zu der vorbestimmten Richtung geschwenkt wird.

## Revendications

1. Dispositif de suture (1) comprenant :
une unité de gaine (3) ayant un premier élément long (31) et un second élément long (32), chacun ayant une extrémité distale et une extrémité proximale, le second élément long (32) étant prévu pour pouvoir coulisser dans une direction axiale par rapport au premier élément long (31) ;
une unité de suture (5) prévue au niveau d'une extrémité distale de l'unité de gaine (3) ; et
une unité d'actionnement (4) prévue au niveau d'une extrémité proximale de l'unité de gaine (3), dans lequel :
l'unité de suture (5) comprend :
un premier bras (51) raccordé à l'extrémité distale du premier élément long (31) et ayant une partie de support de fil (51a) prévue avec une partie de logement d'élément de mise en prise (51f) configurée pour loger, de manière amovible, un élément de mise en prise (6a, 6b) prévu au niveau d'une partie d'extrémité d'un fil de suture, et
un second bras (52) ayant un élément en forme d'aiguille (52a), prévu afin de pouvoir coulisser par rapport au premier bras (51), et raccordé à l'extrémité distale du second élément long (32), et
l'unité d'actionnement (4) comprend :
une première partie d'actionnement (41) raccordée à l'extrémité proximale du premier élément long (31), et
une seconde partie d'actionnement (42) raccordée à l'extrémité proximale du second élément long (32) et prévue afin de pouvoir coulisser par rapport à la première partie d'actionnement (41), **caractérisé en ce que** :
l'unité d'actionnement (4) comprend en outre un moyen de régulation de coulissement (41d, 42d, 43, 43') (41h, 42h, 44) (45, 42i) pour fixer, de manière amovible, la première partie d'actionnement (41) et la seconde partie d'actionnement (42) dans un état dans lequel la première partie d'actionnement (41) et la seconde partie d'actionnement (42) sont disposées de sorte qu'un état arrêté se produit, dans lequel une partie d'extrémité distale (52a4) de l'élément en forme d'aiguille (52a) est dans la partie de logement d'élément de mise en prise (51f) ou dans une position dans laquelle la distance par rapport à une surface au niveau de l'extrémité proximale de la partie de support de fil (51a) est de 3 mm ou moins sans mise en prise avec l'élément de mise en prise (6a, 6b).

2. Dispositif de suture (1) selon la revendication 1, dans lequel :
le moyen de régulation de coulissement (41d, 42d, 43, 43') comprend une première partie de saillie (41d) prévue sur la première partie d'actionnement (41), une seconde partie de saillie (42d) prévue sur la seconde partie d'actionnement (42) afin de correspondre à la première partie en saillie (41d), et un élément de fixation (43, 43'), et
l'élément de fixation (43, 43') comprend une première partie montée (43b, 43b') ayant une partie d'extrémité dans laquelle une partie de la première partie de saillie (41d) peut être montée, de manière détachable, à l'état arrêté, une seconde partie montée (43c, 43c') ayant l'autre partie d'extrémité dans laquelle une partie de la seconde partie de saillie (42d) peut être fixée, de manière détachable, à l'état arrêté, et une partie de raccordement (43a, 43a') raccordant, de manière solidaire, la première partie montée (43b, 43b') et la seconde partie montée (43c, 43c').

3. Dispositif de suture (1) selon la revendication 1, dans lequel :
le moyen de régulation de coulissement (41h, 42h, 44) comprend une première partie de trou débouchant (41h) prévue dans la première partie d'actionnement (41), une seconde partie de trou débouchant (42h) prévue dans la seconde partie d'actionnement (42) afin de correspondre à la première partie de trou débouchant (41h) à l'état arrêté, et un élément de broche (44) pouvant être inséré d'une manière insérable et amovible dans la première partie de trou débouchant (41h) et la seconde partie de trou débouchant dans un état dans lequel la première partie de trou débouchant et la seconde partie de trou débouchant (42h) correspondent l'une à l'autre.

4. Dispositif de suture (1) selon la revendication 1, dans lequel :
le moyen de régulation de coulissement (45, 42i) comprend un élément d'attache (45) prévu, de manière pivotante, sur l'une parmi la première partie d'actionnement (41) et la seconde partie d'actionnement (42) et une partie mise en prise (42i) prévue dans l'autre parmi la première partie d'actionnement (41) et la seconde partie d'actionnement (42), une partie d'extrémité distale (45b) de l'élément d'attache (45) pouvant être mise en prise, de manière amovible, avec la partie mise en prise (42i), et
la partie d'extrémité distale (45b) de l'élément d'attache (45) peut se mettre en prise avec la partie mise en prise (42i) par l'élément d'attache (45) qui est pivoté dans une direction prédéterminée à l'état arrêté et la mise en prise de la partie d'extrémité distale (45b) de l'élément d'attache (45) avec la partie mise en prise (42i) peut être libérée par l'élément d'attache (45) qui est pivoté dans une direction opposée à la direction prédéterminée.
